# EUROPEAN PATENT APPLICATION

(11) **EP 0 732 399 A2**
(43) Date of publication of application: **18.09.1996**
(21) Application number: 96103933.6
(22) Date of filing: 04.03.1991
(51) Int. Cl.: C12N 9/64, C12Q 1/37, C07K 5/00, C07K 7/00, A61K 38/04

(54) **Chymotrypsin-like proteases and their inhibitors**

(30) Priority: 05.03.1990 US 489290
(62) Divisional of application: 91905743.0
(71) Applicant: CEPHALON, INC., West Chester, PA 19380 (US)
(72) Inventor: Siman, Robert, Wilmington, Delaware 19810 (US); Nelson, Robert B., N. Chelmsford, New Hampshire 01863 (US); Kauer, James C., Kennett Square, Pennsylvania 19348 (US); Potter, Huntington, Boston, Massachusetts 02215 (US)
(74) Representative: Sheard, Andrew Gregory

(57) **Abstract**

Methods are disclosed for the detection of proteases indicative of Alzheimer's disease which are capable of cleaving β-amyloid precursor protein between methionine and aspartic acid residues. Inhibitors and substrates for these enzymes are also described which are used in the preparation of agents for the treatment of the disease.

## Description

### Background of the Invention

This invention relates to chymotrypsin-like proteases, and substrates and inhibitors thereof, involved in the etiology of Alzheimer's disease.

Alzheimer's disease (AD) is a progressive degenerative disorder of the brain that afflicts over four million people in the United States. No effective treatment is presently available. AD is only definitively diagnosed by post-mortem histopathological methods. Even in its end-stage, AD is sometimes confused with disorders that manifest dementia, such as depression, which can be treated if correctly diagnosed. Thus, there is an enormous need for strategies to develop AD diagnostics and therapeutics.

An invariant feature of AD neuropathology is the deposition of protein, known as beta-amyloid or A4, into neuritic and cerebrovascular plagues. The presence of dense beta-amyloid plaques in large areas of the cerebral cortex is diagnostic for AD, according to guidelines of the National Institute on Aging (Khachaturian, Arch. Neurol. 42:1097, 1985). Beta-amyloid is a 39-42 amino acid peptide (Glenner et al., Biochem. Biophys. Res. Commun. 120:885, 1984; Masters et al., Proc. Natl. Acad. Sci. 82:4245, 1985). It is synthesized as part of larger precursor proteins (Kang et al., Nature 325:733, 1987; Tanzi et al., Nature 331:528, 1988; Ponte et al., Nature 331:525, 1988; Kitaguchi et al., Nature 331:530, 1988). Proteolytic processing of these beta-amyloid precursor proteins (APP) is required to generate beta-amyloid. The amino terminal of beta-amyloid is generated by cleavage of the peptide bond in APP between Met⁵⁹⁶ and Asp⁵⁹⁷ (numbering according to Kang et al., supra).

Abraham et al. (Biotechnology 7:147, 1989) state that "it is interesting to note that the N-terminal of the two cleavage sites within the precursor of the beta-protein contains a methionine and could therefore result from processing by a chymotrypsin-like protease". Similarly, Van Nostrand et al. (Nature 341:546, 1989) state that "several findings have implicated the involvement of a chymotrypsin-like protease in the deposition of amyloid beta-protein in Alzheimer's disease neuritic plaques". Abraham (Neurobiol. Aging 10:463, 1989) states that "protease inhibitors are, therefore, attractive targets for drug intervention in Alzheimer's disease".

Pope et al. (Neurochem. Res. 9:291, 1984) describe proteases found in the human brain. They state that the neuritic plaques that characterize AD are the result of excess protein accumulation consistent with impaired steady-state proteolysis and/or protein quality control. They suggest that it is likely "that comprehensive analysis of neural proteolytic machinery will be fruitful for improved understanding of the molecular and structural lesions in the cerebrospinal abiotrophies".

Among the proteases identified in mammalian brain are two serine proteases that have chymotrypsin-like activity. One is an enzyme complex referred to as "ingensin" (Ishiura et al., FEBS Lett. 189:119, 1985) or the "multicatalytic protease" (Wilk et al., J. Neurochem. 40:842, 1983). This protease complex contains chymotrypsin-like activity, in that it cleaves peptide bonds on the carboxy terminal side of aromatic or large aliphatic amino acids. Secondly, mast cells have been found in the brains of several mammals (Dropp, Acta Anat. 94:1, 1976); these cells contain chymotrypsin-like protease (Woodbury et al., Proc. Natl. Acad. Sci. 75:5311, 1978; Schechter et al., J. Immunol. 137:962, 1986).

There are a number of publications concerning inhibitors for proteases and, in particular, for serine proteases. These include: Nakajima et al., J. Biol. Chem. 254:4027, 1979; Imperiali et al., Biochemistry 25:3760, 1986; Kettner et al., J. Biol. Chem. 259:15106, 1984; Kettner et al., U.S. Patents 4,582,821, 4,499,082, 4,644,055, 4,636,492, 4,652,552, and EPA 0,187,721.

The following abbreviations are used for amino acids and amino acid residues:

| AMINO ACID | ABBREVIATION | ONE LETTER CODE |
|---|---|---|
| L-alanine | Ala | A |
| L-arginine | Arg | R |
| L-asparagine | Asn | N |
| L-aspartic acid | Asp | D |
| L-cysteine | Cys | C |
| L-glutamine | Gln | Q |
| L-glutamic acid | Glu | E |
| Glycine | Gly | G |
| L-histidine | His | H |
| L-isoleucine | Ile | I |
| L-leucine | Leu | L |
| L-lysine | Lys | K |
| L-methionine | Met | M |
| L-phenylalanine | Phe | F |
| L-proline | Pro | P |
| L-serine | Ser | S |
| L-threonine | Thr | T |
| L-tryptophan | Trp | W |
| L-tyrosine | Tyr | Y |
| L-valine | Val | V |
| L-ornithine | Orn | |
| L-norleucine | Nle | |
| L-homoarginine | Harg | |
| L-citrulline | Cit | |
| L-norvaline | Nva | |
| L-pyroglutamic acid | Pglu | |

Where prefixed by "D-", the foregoing abbreviations indicate an amino acid of D-configuration.

The following abbreviations are used for N-terminal blocking groups:
- Boc: t-Butyloxycarbonyl
- Ac: Acetyl
- Et: Ethyl
- Suc: Succinyl
- MeOSuc: Methoxysuccinyl
- Sub: Suberyl
- Ad: Adipyl
- Az: Azelayl
- DNS: Dansyl
- DNP: 2,4-Dinitrophenyl
- Z: Benzyloxycarbonyl
- Fmoc: Fluorenylmethoxycarbonyl
- MeOAz: Methoxyazelayl
- MeOAd: Methoxyadipyl
- MeOSub: Methoxysuberyl

### Summary of Invention

The present invention concerns the identification, characterization, and purification of two chymotrypsin-like serine proteases characteristic of AD, called "chymase" and "multicatalytic protease", that are derived from rat or human brain. Both chymase and the multicatalytic protease have an enzymatic activity capable of cleaving between Met and Asp, which activity is required to generate beta-amyloid. Methods are provided for quantifying the activities of chymase and the multicatalytic protease in brain (and other tissue samples and fluids) to allow diagnosis of AD, and for development of inhibitors as therapeutic agents to slow progress of the disease. Novel compositions have been prepared and identified as inhibitors of the two enzymes. Methods are also provided for cloning and sequencing of human brain chymase and the multicatalytic protease, and the preparation of nucleic acid and antibody probes to them.

Thus, in a first aspect the invention features a purified peptide (which term includes polypeptides and proteins) having an endopeptidase enzymatic activity which causes hydrolysis of a peptide bond between a methionine and an aspartic acid in a beta-amyloid precursor protein. By "purified" is meant that the peptide is separated from components of a cell or tissue in which it naturally occurs; preferably it is purified to represent at least 70% by weight, most preferably 90%, of the peptides present in a solution or preparation.

In a second aspect, the invention features a method for detection in a biological sample of an protease indicative of Alzheimer's disease. The method includes providing a substrate for the protease having the formula I:

R-A4-A3-A2-A1-R1 I:

wherein R is an N-terminal blocking group or hydrogen; R1 is a reporter group; A4 is a covalent bond (i.e., R is directly bonded to A3), an amino acid or a peptide, e.g., including up to about five D- or L-amino acids; A3 a covalent bond (i.e., A4 is directly bonded to A2 or when A4 is a covalent bond R is directly bonded to A2), or is a D-amino acid, Phe Tyr, or Val or a conservative amino acid substituent of Val, e.g., Leu, Ile, Nle, Ala, and Nva; A2 is a hydrophobic amino acid, e.g., Leu, Ile, Nle, Phe, Val, Nva, Tyr, or Pro, or preferably lysine or a conservative amino acid substituent thereof, e.g., Arg, Orn, Cit, and Harg or when A4 includes at least two amino acids, A2 is any amino acid; and A1 is Met, Phe, Nle, Leu, Ile, Tyr, or 3-phenylproline; when A4 and/or A3 are a covalent bond, R may be an alkane dicarboxylic acid or alkane carboxylic acid of 2 to 20 carbon atoms, or a lower alkyl monoester thereof, optionally substituted by an alkyl, aralkyl, or aryl substituent; preferred substituents are isopropyl, isobutyl, benzyl, or phenyl, and preferred lengths are 3 to 10 carbon atoms. The method also includes contacting the biological sample with the substrate, and detecting cleavage of the substrate as an indication of the presence of the protease in the biological sample.

R1 is a reporter group capable of forming an amide or ester linkage with the C-terminal carbonyl of amino acid residue A1 and which, on release from the peptide sequence by the cleavage reaction, forms a molecule R1-H or an anion R1⁻ which may be detected by visual or instrumental means.

The precise nature of the reporter group R1 is not critical to the present invention. By "reporter group" is meant any chromogenic, magnetic, fluorogenic, luminescent, antigenic, stable free radical or radioactive group which is detectable by any one or more of the techniques used for detecting particular analytes when in solution, or attached to a suitable surface.

When R1 is a chromogenic group, it forms a covalent molecule R1-H after cleavage, or a covalent ion R1⁻, which may optionally be separated from the unreacted peptide by solvent extraction, precipitation, or other chemical or physical means which allows the free R1-H or R¹⁻ to be determined visually or instrumentally. For example, when R1-H is p-nitroaniline (R1⁻ is a p-nitroanilide (pNa) ion), it may be determined quantitatively by its ultraviolet or visible light absorption in a photometer, or visually be comparison with a series of standard solutions. Other suitable chromogenic groups include nitronaphthyloxy, naphthylamino, nitronaphthylamino, and quinolinamino.

When R1 is a fluorogenic group, the free R1⁻ or R1-H may be detected in solution by exposing the solution to light of one wavelength and detecting fluorescence at another, usually longer, wavelength. Suitable fluorogenic groups include 4-methylcoumarin-7-amino (AMC), 2-naphthylamino, 4-trifluoromethylcoumarin-7-amino, and 4-methylumbelliferyl.

R1-H may be measured quantitatively by nuclear magnetic resonance (NMR) by incorporating a substituent with a magnetically distinctive nucleus which produces a sharp resonance detection, and a fluoroalkyl or fluoroaryl substituent for 19F magnetic resonance detection.

When R1 is a stable free radical (e.g., nitroxyl or diphenylpicrylhydrazyl) substituent this permits its detection by electron spin resonance (ESR).

If cleavage of R1 from the peptide is accompanied by a marked change in the physical properties of the R1 group, e.g., a significant change in the intensity or wavelength of the ultraviolet absorption maximum, the physical separation of the released substituent (R1⁻ or R1-H) from the unreacted substrate may optionally be omitted. The amount of cleavage is determined quantitatively by measurement of the absorption of the released substituent R1-, at or near its ultraviolet or visible light absorption maximum.

By "N-terminal blocking group" is meant a D-amino acid or an arylcarbonyl, alkylcarbonyl, alkoxycarbonyl, aryloxycarbonyl, aralkyloxycarbonyl, aralkylsulfonyl, alkylsulfonyl, or arylsulfonyl peptide protecting group, or other equivalents known to those skilled in the art of peptide synthesis and which are known to protect molecules from degradation by aminopeptidases (Gross and Meienhofer, eds., The Peptides, vol. 3, Academic Press, New York, 1981 pp. 3-81, describes numerous suitable amine protecting groups). As used herein, either individually or as part of a larger group, "alkyl" means a linear, cyclic, or branched-chain aliphatic moiety of 1 to 20 carbon atoms; "aryl" means an aromatic moiety, e.g., phenyl, of 6 to 18 carbon atoms, unsubstituted or substituted with one or more alkyl, substituted alkyl, nitro, alkoxy, or halo groups; "substituted alkyl" means an alkyl group having a substituent containing a heteroatom or heteroatoms such as N, O or S; "halo" means Cl or Br, and "alkaryl" means an aryl moiety of 7 to 19 carbons having an aliphatic substituent, and, optionally, other substiuents such as one or more alkyl, substituted alkyl, alkoxy or amino groups. "Aralkyl" means a linear or branched chain aliphatic moiety of 7 to 18 carbon atoms including an aryl group or groups.

Examples of suitable N-terminal blocking groups include formyl, acetyl, trifluoroacetyl, benzyloxycarbonyl (carbobenzyloxy), substituted benzyloxycarbonyl, tertiary butyloxycarbonyl, isopropyloxycarbonyl, allyloxycarbonyl, phthaloyl, benzoyl, acetoacetyl, chloroacetyl, phenoxycarbonyl, methoxysuccinyl, succinyl, adipyl, suberyl, 2,4-dinitrophenyl, dansyl, p-methoxybenzenesulfonyl, p-toluenesulfonyl, methanesulfonyl, D-serine, and D-glutamic acid.

In preferred embodiments, the biological sample is isolated from brain, liver, heart, muscle, white blood cells, mast cells or fibroblasts, serum or cerebrospinal fluid; the protease is chymase; the protease is multicatalytic protease.

In a third aspect, the invention features a substrate for a protease, having the formula I described above wherein R is an N-terminal blocking group or hydrogen; R1 is a reporter group; A4 is a covalent bond (i.e., R is directly bonded to A3), an amino acid or a peptide, e.g., including up to about five D- or L-amino acids; A3 a covalent bond (i.e., A4 is directly bonded to A2 or when A4 is a covalent bond R is directly bonded to A2), or is a D-amino acid, Phe Tyr, or Val or a conservative amino acid substituent of val, e.g., Leu, Ile, Nle, Ala, and Nva; A2 is a hydrophobic amino acid, e.g., Leu, Ile, Nle, Phe, Val, Nva, Tyr, or Pro, or preferably lysine or a conservative amino acid substituent thereof, e.g., Arg, Orn, Cit, and Harg or when A4 includes at least two amino acids, A2 is any amino acid; and A1 is Met, Phe, Nle, Leu, Ile, Tyr, or 3-phenylproline; when A4 and/or A3 are a covalent bond, R may be an alkane dicarboxylic acid or alkane carboxylic acid of 2 to 20 carbon atoms, or a lower alkyl monoester thereof, optionally substituted by an alkyl, aralkyl, or aryl substituent; preferred substituents are isopropyl, isobutyl, benzyl, or phenyl, and preferred lengths are 3 to 10 carbon atoms. In preferred embodiments, A3 is Val, Nle, Nva, Ile, Leu, Tyr, or Phe; A2 is Lys, Arg, Orn, Cit, or Harg; A4 is Glu or Asp; R is Boc, Fmoc, Ac, Et, Suc, MeOSuc, Az, MeOAz, Ad, MeOAd, Sub, MeOSub, DNS, DNP, Z, or a D-amino acid; and R2 is pNa or AMC.

In a fourth aspect, the invention features an inhibitor of a protease, having the formula II:

R-A4-A3-A2-Y II:

where R, A4, A3, and A2 are as described above, and Y is a group reactive with the active site of the protease. These inhibitors have therapeutic utility derived from their ability to inhibit the enzyme "chymase" or the enzyme "multicatalytic protease" or related enzymes. These inhibitors include peptide sequences protected at their N-terminus by a protecting group, and attached at their C-terminus by a covalent bond to a moiety Y, which interacts strongly with functional groups located near the reactive site of the enzyme.

The N-terminal blocking group provides metabolic stability to the composition by reducing or eliminating its ability to be degraded by endogenous aminopeptidases. The carboxyl terminal moiety Y allows a strong chemical interaction, mediated by covalent or noncovalent bond formation, at the active site of the enzyme to effectively inactivate the enzyme.

Y is a group which strongly interacts with functional groups in or near the enzyme active site and is covalently attached to the carbonyl group of amino acid A2 by an amide bond. It is provided with a lipophilic side chain substituent R2, which interacts with the lipophilic substituent recognition site of the enzyme to be inhibited, e.g., chymase or the multicatalytic protease, and with a functional group R3, which can react with or interact with an adjoining serine hydroxyl group to form a transition-state analog (Rich, Protease Inhibitors, ed. Barrett and Salversen, Elsevier, 1986, p. 167.

Y may be derived from an amino acid analog Y-H in which Y has the formula III: 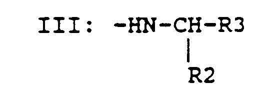 wherein R2 may be a saturated or unstaturated alkyl or substituted alkyl group of 2 to 10 carbon atoms, alicyclic or aromatic groups of 5 to 10 carbon atoms or arylalkyl groups of up to 11 carbon atoms. Examples of suitable R2 groups include ethyl, n-propyl, isopropyl, n-butyl, sec-butyl, isobutyl n-pentyl, n-hexyl, isohexyl, n-decyl, phenyl, benzyl, beta-phenylethyl, alpha-naphthyl, beta-naphthylmethyl, cyclohexyl, cyclohexylmethyl, adamantyl, alpha-styryl, beta-styryl, and propargyl.

Examples of suitable R3 groups include the aldehyde and ketone functions -CHO, -CO-CH₃, -CO-CH₂Cl, -CO-CH₂Br, and boronic acid residues, e.g., -B-(OH)₂, 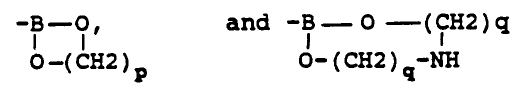 where p and q are 2 or 3; as well as alphadiketones, e.g., 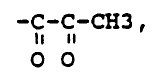 and alphaketoesters, e.g.,

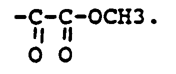

Other suitable R3 groups include -SO₂F, -C₆H₄-SO₂F, and -PO(OR4)F where R4 is a lower alkyl or aralkyl substituent.

Additional suitable Y groups include the acylating heterocyclic ring systems described by Powers and Harper, in Proteinase Inhibitors, A.J.Barrett and G. Salversen, eds., Elservier, New York, 1986, pp. 108-132. These substituents generally interact with the enzyme active-site serine and may subsequently react with other adjoining functionalities such as histidine side chains. Examples of such Y groups include N-substituted saccharins, as well as derivatives of benzoxazin-4-ones, 3-alkoxy-4-chloroisocoumarins, oxazine-2,6-diones, substituted isatoic acids, halomethylcoumarins, N-nitrosoisoquinolinones, haloenol lactones, isobenzofuranones, ynenol tetrahydro-2-furanones, 2-pyranones, chloropyrones, chloroisocoumarins and the like.

Other Y groups may be derived from alpha-aza amino acids and their amide, ester, and peptide derivatives of formula IV: 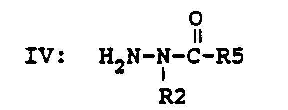 where R2 is as defined in formula III and R5 may be derived respectively from an amine or ammonia, an alcohol, or a peptide sequence substituted at its amino terminus. Examples of the synthesis of such aza amino acids and azapeptides are reviewed by J. Gante, Synthesis, 405-413, 1989. Preferred values of R5 are amino acids or peptide sequences found at the amino terminus of beta-amyloid, e.g., asp, asp-ala, asp-ala-glu, asp-ala-glu-phe and their esters and amides.

In preferred embodiments, Y has the formula: 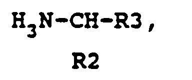 where R2 is a lipophilic side chain substituent and R3 is a functional group which reacts with a hydroxyl group of the protease to form a transition-state analog; R2 is a saturated or unsaturated alkyl or substituted alkyl group which includes two to ten carbon atoms or an alicyclic or aromatic group comprising five to ten carbon atoms or an arylalkyl group which includes eleven or fewer carbon atoms; R2 is an ethyl, n-propyl, isopropyl, n-butyl, or sec-butyl group; R3 is -CHO, -COCH₃, COCH₂Cl, or COCH₂Br; the protease is chymase; the protease is mutlicatalytic protease.

In a fifth aspect, the invention features methods for purifying a protease to at least 70%, preferably 90% or greater, purity from a biological sample. One method includes homogenizing the biological sample, e.g., a biological sample from a mammal such as from human, rat, cow, or pig, in a low salt buffer including detergent; isolating the particulate fraction from the homogenized tissue; dissolving the particulate fraction in a high salt buffer; separating the soluble fraction from the high salt buffer; binding the protease to, and then eluting it from, an ion exchange or a heparin column; and binding the protease, and then eluting it from, an affinity column.

In preferred embodiments, the biological sample is isolated from brain, liver, heart, skeletal muscle, white blood cells, mast cells or fibroblasts; the low salt includes less than 0.5M salt; the detergent is ionic or nonionic, e.g., Triton X-100; the particulate fraction is separated from the homogenized tissue by centrifugation, e.g., at between 30,000 to 300,000 x g for 15 to 120 minutes; the high salt is greater than 0.5M salt; the soluble fraction is separated from the high salt by centrifugation, e.g., at 30,000 to 300,000 x g for 15 to 120 minutes; the protease is bound to a heparin column; the protease is chymase; the protease is multicatalytic protease; and the affinity column includes a substrate for the enzyme. In this affinity cloumn the amino terminus of the substrate is attached to the carrier column material by a covalent bond, preferably by an amide bond; most preferably the substrate has the formula V:

W-X-Y-Z V:

where W is deleted, an amino acid or a peptide, e.g., including up to about five D- or L-amino acids; X is deleted, or is Phe, or is Val or a conservative amino acid substituent of Val, e.g., Leu, Ile, Nle, Ala, and Nva; Y is a hydrophobic amino acid, e.g., Leu, Ile, Nle, Phe, Val, Nva, Pro, or Lys or a conservative amino acid substituent of Lys, e.g., Arg, Orn, Cit, and Harg, or, when W includes at least two amino acids, Y may be any amino acid; and Z is Met, Phe, Nle, Leu, Ile, Tyr or 3-phenylproline.

Alternatively, when W is deleted, X may be a deleted or may be an omega-aminoalkanecarboxylic acid of 2 to 20 carbon atoms in chain length, optionally substituted at the alpha or beta position by an alkyl, aralkyl, or aryl substituent. Preferred substituents are isopropyl, isobutyl, benzyl, and phenyl, and preferred chain lengths are 2-8 carbon atoms.

The second method includes homogenizing the tissue in a low salt buffer; isolating the soluble fraction from the homogenized tissue; binding the protease to, and then eluting it from, an ion exchange or heparin column; concentrating the protease and passing it through a gel filtration column; binding the protease, and then eluting it from, an hydroxyapatite or heparin column.

In a sixth aspect, the invention features a method for treating Alzheimer's disease. The method includes providing an inhibitor of formula II as described above, and administering the inhibitor to a patient in a pharmaceutically acceptable carrier.

In preferred embodiments, the administration is directly into the brain; the administration is intramuscular, oral, or intranasal; the protease inhibitor is an inhibitor of chymase; the protease inhibitor is an inhibitor of multicatalytic protease.

In a seventh aspect, the invention features a method for screening candidate chymase inhibitors. The method includes the steps of: (a) incubating a chymase substrate (e.g., a substrate corresponding to formula I above) with one or more candidate chymase inhibitors and chymase; and (b) determining whether the one or more candidate chymase inhibitors decrease the rate of cleavage of the substrate by the chymase.

In a eighth aspect, the invention features a method for screening candidate multicatalytic protease inhibitors. The method includes the steps of: (a) incubating a multicatalytic protease substrate (e.g., a substrate corresponding to formula I above) with one or more candidate multicatalytic protease and multicatalytic; and (b) determining whether the one or more candidate multicatalytic protease inhibitors decrease the rate of cleavage of the substrate by the multicatalytic protease.

In an eighth aspect, the invention features a method for screening candidate proteases capable of cleaving a beta-amyloid precursor protein between Met and Asp. The method includes of: (a) providing a peptide having the sequence: EVKMDAEFRHDSGYEVHHQ; (b) contacting the candidate protease with the peptide; and (c) determining whether the candidate protease cleaves the substrate.

In a ninth aspect, the invention features a method for screening proteases capable of cleaving a beta-amyloid precursor protein between Met and Asp. The method includes the steps of (a) providing a substrate having the sequence R-Glu-Val-Lys-Met-R1, wherein R is hydrogen or an N-terminal blocking group, R1 is a reporter gorup; (b) contacting the candidate protease with the substrate; and (c) determining whether said candidate protease cleaves the substrate.

In a tenth aspect, the invention features a method for treating a patient afflicted with Alzheimer's disease. The method includes administering to the patient an inhibitor of chymase in a pharmaceutically acceptable carrier.

In an eleventh aspect, the invention features a method for treating a patient afflicted with Alzheimer's disease. The method includes administering to the patient an inhibitor of multicatalytic protease in a pharmaceutically acceptable carrier.

By "inhibitor of chymase" is meant a molecule capable of decreasing the ability of chymase to catalyze proteolytic cleavage of a protein, particularly proteolytic cleavage a beta-amyloid precursor protein.

By "inhibitor of multicatalytic protease" is meant a molecule capable of decreasing the ability of chymase to catalyze proteolytic cleavage of a protein, particularly proteolytic cleavage of a beta-amyloid precursor protein.

This invention provides enzymes able to cleave a Met-Asp bond in an APP. The quantity of such enzymes in tissue is diagnostic of AD; their detection allows a rapid, simple, and non-invasive assay for AD. Such detection may be by enzymatic test, Western blot, or Southern or Northern blotting techniques. Further, inhibition of the enzyme activity *in vivo* allows progression of AD to be slowed. Such inhibition is caused by the above described inhibitors, and by antibodies to the enzymes.

Other features and advantages of the invention will be apparent from the following description of the preferred embodiments thereof, and from the claims.

### Description of the Preferred Embodiments

The drawings will first briefly be described.

### Drawings

Fig. 1 depicts an SDS-polyacrylamide gel analysis of purified chymase;
Fig. 2 depicts the results of the purification scheme for brain chymase;
Fig. 3 is a depiction of a comparison of the partial amino acid sequence of purified brain chymase (Brain) and the partial amino acid sequence of rat mast cell protease I (RMCP I);
Fig. 4 is a set of graphs which depict the results of column chromatography used to purify chymase;
Fig. 5 is a schematic illustration of the beta/A4 protein and the region of beta/A4 protein used in design protease substrates;
Fig. 6 is a set of graphs which depict the degradation of substrates brain chymase (panel A) chymotrypsin (panel B), and cathepsin G (panel C);
Fig. 7 is a set of graphs which illustrate the results of HPLC analysis of peptides generated by chymase degradation;
Fig. 8 depicts an SDS-polyacrylamide gel analysis purified chymase;
Fig. 9 depicts the results of a purification scheme for chymase;
Fig. 10 depicts the results of column chromatography used to purify chymase;
Fig. 11 depicts the results of kinetic analysis of degradation of substrates by rat brain chymase;
Fig. 12 is a set of graphs which depict the inhibition of protease degradation by inhibitors;
Fig. 13 depicts inhibition of various proteases by inhibitors;
Fig. 14 depicts the effect of inhibitors of degradation of substrates by a protease;
Fig. 15 is a graphical representation of protease activity in various parts of a Alzheimer's disease brain;
Fig. 16 depicts the structures of a number of protease inhibitors.

### Substrates and Inhibitors

Substrates useful in this invention have the general formula I described above. They can be used to measure the level of chymotrypsin-like proteases characteristic of Alzheimer's disease in body tissues and fluids. These compositions are peptide sequences preferably protected at their amino termini by a blocking group and attached at the carboxy termini by an amide or ester bond to a reporter group which is released when the bond is cleaved by the protease. Such peptide compositions also include salts of the above general formulae. Generally, the amino acid analogs are chosen such that their identity and sequence substantially corresponds to, or is identical to, those amino acids located immediately adjacent to and upstream of a cleavage recognition site of the enzymes chymase or the multicatalytic protease in an APP.

By "upstream" is meant the direction which is the reverse of the direction of translation, i.e., toward the amino terminus of a peptide.

By "substantially corresponds" is meant that a peptide sequence comprises a like number of amino acid residues as a reference sequence, the identity and sequence of which are exactly homologous, or conservatively substituted, in relation to the reference sequence. By "conservatively substituted" is meant that a given amino acid residue is replaced by a biologically similar residue. Examples of conservative substitution include substitution of one alkyl or substituted alkyl residue for another such as Nle, Met, Ile, Val, Leu, Phe or Tyr for one another, or substitution of one polar residue for another, such as Lys and Arg; Glu and Asp; or Gln and Asn. "Residue" means either an amino acid or amino acid analogue.

Acceptable salts of the compounds include the salts of free peptide acids including sodium, potassium, magnesium, calcium, and ammonium salts as well as acid addition salts of the free peptide bases including both inorganic and organic acids such as hydrochloric, sulfuric, phosphoric, acetic, citric, or trifluoroacetic acids.

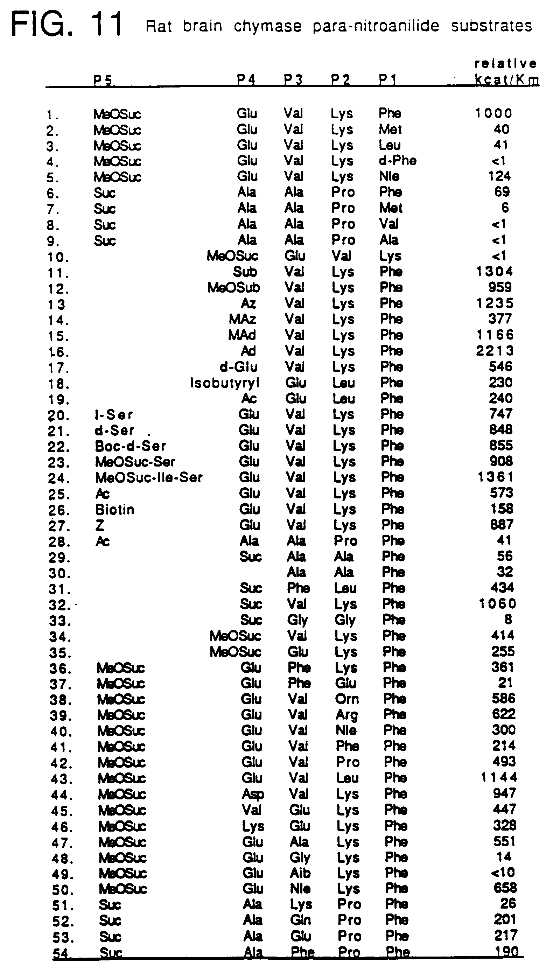

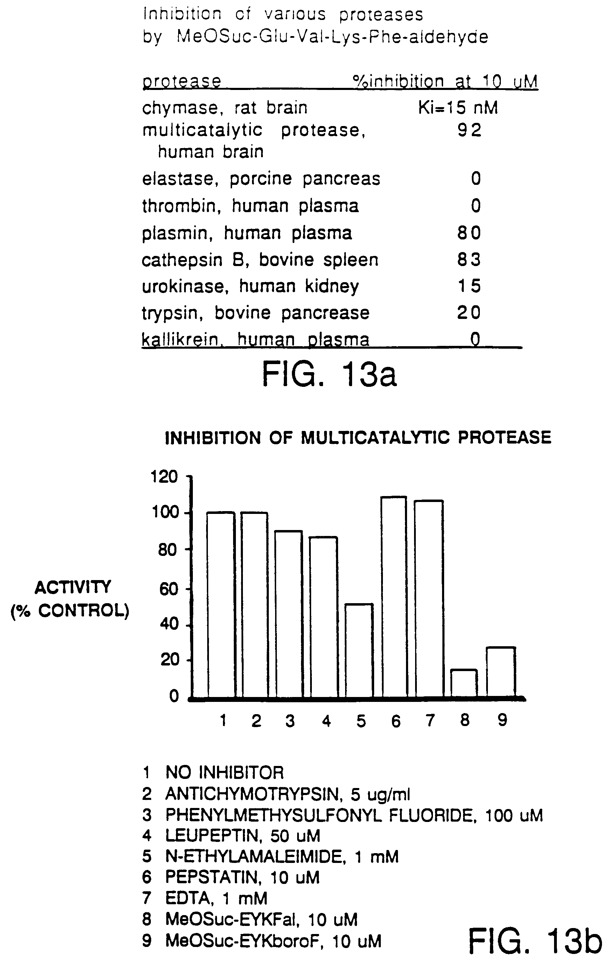

Preferred compounds of this invention include peptide derivatives of formula I in which A1 is selected from the group Phe, Met, Leu, Ile, Nle, Tyr, or 3-phenylproline; A2 is selected from the group Lys, Arg, Orn, Cit, or Harg; A3 is selected from the group Val, Leu, Ile, Nle, Phe, Nva, Pro, or is a D-amino acid, or a covalent bond (i.e., A4 is bonded directly to A2 or when A4 is a covalent bond R is bonded directly to A2); A4 is Glu, Asp, D-Glu or D-Asp, or is a covalent bond (i.e., R is bonded directly to A3); R is one of the protecting groups previously described or a D-amino acid; and R1 is selected from pNa and AMC. Specific examples of such compounds are shown in Figures 11 and 13.

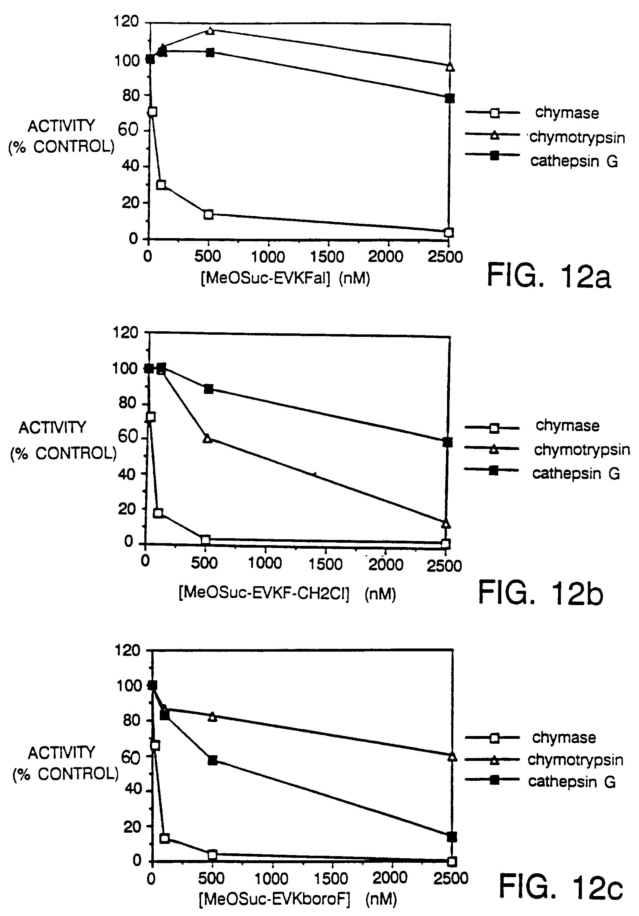

Inhibitors have the general formula II described above. These are designed in a manner similar to the substrates but have a reactive C-terminal group as described above. Specific examples of such inhibitors include those shown in Figures 12, 13 and 16.

The above compositions can be used to measure the levels of the enzymes chymase and the multicatalytic protease in body tissues and fluids and to inhibit their activity. These compositions are generally peptide or peptidomimetic sequences protected at the amino terminus by a blocking group. These compounds can be prepared by the reaction of a precursor peptide derivative, for example R-A4-A3-A2-OH with the amino acid derivatives H-A1-R1 or H-Y respectively.

The peptide precursors may be prepared by well-known synthetic procedures using either the solution phase techniques described in "The Peptides: Analysis, Synthesis, Biology" Volume I (1979), eds. Gross et al. (eds.), Academic Press, or the solid phase techniques described in Volume II (1980) of the above series.

The peptide precursors R-A4-A3-A2-OH with protecting groups in place may be prepared by the solid phase method using the support resin described by Mergler et al., Tetrahedron Lett. 29:4009 (1988) and commercially available under the name "Sasrin" from Bachem Biosciences, Inc., Philadelphia, PA. 19104. The peptide sequences are built up on the resin by sequential treatment of the resin with amino acid derivatives beginning with the fluorenylmethoxycarbonyl derivative of amino acid A2 (Fmoc-A2), in the presence of a coupling reagent such as benzotriazolyloxytris (dimethylamino) phosphonium hexafluorophosphate (BOP), followed by treatment with piperidine to remove the Fmoc protecting group as described by Atherton et al., J. Chem. Soc. Perkin Transactions I, 2057 (1985). The use of the BOP reagent is described by Castro et al., Synthesis, 751-2 (1976).

Subsequently the Fmoc derivative of A3 and the corresponding components of R-A4 (see below) are added with Fmoc deblocking by piperidine between successive amino acid additions. The functional side chains of the amino acids A2, A3, and A4 are protected as follows: lysine is protected by the t-butoxycarbonyl (Boc) group; histidine and cysteine are protected by the N-triphenylmethyl (trityl) group; arginine is protected with the 4-methoxy-2,3,6-trimethylphenylsulfonyl (Mtr) group; tyrosine, serine, and threonine are protected as the t-butyl ethers (t-Bu); and glutamic and aspartic acids are protected as the t-butyl esters (Ot-Bu). These particular protecting groups remain on the peptide-resin complex throughout the solid phase synthesis of peptide R-A4-A3-A2 on the resin as well as during the cleavage of the peptide precursor R-A4-A3-A2-OH from the resin with a dilute solution of trifluoroacetic acid (TFA) in methylene chloride.

Alternatively, this peptide precursor may be built up by solution phase methods using the strategies described in Gross and Meienhofer, supra (volume I). The solution phase strategy is particularly well suited to shorter peptides in which A4 is one or two amino acids. Thus, where A4 is a single amino acid, a peptide is built up by reacting the Fmoc derivative of A4 as a N-hydroxysuccinimide ester (Fmoc-A4-OSu) with a buffered solution of amino acid A3. The resulting Fmoc-A4-A3-OH can next be reacted with the benzyl ester of amino acid A2 (A2-OBzl) to give Fmoc A4-A3-A2-OBzl. Alternatively, Fmoc-A4-OSu can be reacted directly with the dipeptide ester A3-A2-OBzl to give the same product.

Fmoc-A4-A3-A2-OBzl is next treated with piperidine to remove the Fmoc protecting group, and can be reacted with R-A5 (where A5 is an optional amino acid, see below) in the presence of BOP to give R-A5-A4-A3-A2-OBzl. Alternatively, Fmoc-A3-A2-OBzl can be prepared and reacted to give R-A4-A3-A2-OBzl using this methodology.

The terminal group R-A5 (or R-A4) may be prepared by treatment of the side-chain-protected terminal amino acid group A5-OH with an acylating derivative of R. Thus when R is an acyl (e.g., alkylcarbonyl, arylcarbonyl, alkoxycarbonyl, aryloxycarbonyl, or aralkyloxycarbonyl) or a sulfonyl (e.g., arylsulfonyl, alkylsulfonyl, or aralkylsulfonyl) function, the group R may be attached to the side-chain-protected group A5-OH using the acid chloride of R (R-Cl), or the symmetric anhydride of R (R-OR), or the mixed carbonic anhydride of R (e.g., R-O-COO-isobutyl) or preferably an active ester of R such as its N-hydroxysuccinimide ester in the presence of a pH-buffering agent such as sodium bicarbonate or N-methylmorpholine.

When the N-terminal group R is a D-amino acid it may most conveniently be added to the side-chain-protected amino acid function A5-OH as the Boc derivative of R to give Boc-R-A5-OH. The Boc protecting group is then carried throughout the synthesis of the protected epeptides and is removed at the end by treatment with TFA.

Side-chain-protected Fmoc-A4-A3-A2-OBzl prepared as described above is next deblocked with piperidine to give A4-A3-A2-OBzl which is, in turn, condensed with side-chain-protected R-A5-OH using BOP or diisopropyl carbodiimide to yield R-A5-A4-A3-A2-OBzl. Chemical reduction or catalytic hydrogenation of this latter compound gives the side-chain-protected precursor peptide derivative.

This side-chain-protected precursor peptide is then converted to an active ester or a mixed anhydride (Gross and Meienhofer, I, loc. cit.) which in turn is reacted with H-Y to yield the inhibitors of formula II.

The substrate compounds H-A1-R1 are purchased or are prepared by treatment of an active ester of Boc-A1 with H-R1 followed by treatment of the resulting Boc-A1-R1 with hydrochloric acid or TFA. Thus Boc-Phe hydroxysuccinimide (Fluka Chemical Corp., Ronkonkoma, N.Y.) is treated with p-nitroaniline to give Boc-Phe-p-nitroanilide. Treatment of this with TFA gives Phe-p-nitroanilide.

The compounds H-Y are synthesized by methods well documented in the literature:

Where H-Y is an alpha amino aldehyde (i.e., R3 is CHO), the corresponding methyl, ethyl or butyl acetal is prepared as described by Gacek et al., Tetrahedron 30, 4233 (1974) or by Edwards, European Patent Application 291234 filed May 5, 1988.

Where H-Y is a chloromethyl ketone (i.e., R3 is COCH₂Cl) the hydrochloride salt of the chloromethyl ketone may be purchased (Bachem Bioscience, Inc., Philadelphia, PA.) or can be prepared by conversion of the Boc-protected amino acid to a mixed carbonic anhydride followed by reaction with diazomethan to give diazolktones, then with anhydrous hydrogen chloride (Kettner et al., Arch. Biochem. Biophys. 162:56 (1974) and Fittkau, J. Prakt. Chem. 315:1037, 1973). The corresponding bromomethyl ketones (i.e., R3 is COCH₂Br) may be prepared similarly by the substitution of hydrogen bromide for hydrogen chloride (C. Kettner et al., *supra*). The corresponding methyl ketones are prepared by hydrogenolysis of the chloromethyl ketones with palladium-on-charcoal in methanol (Kettner et al., U.S. Patent 4,652,552).

Where H-Y is a boroamino acid (i.e., R3 is B(OH)₂) derivative, the cyclic ester of the boroamino acid is prepared substantially as described by Shenvi, U.S. Patent 4,537,773 and after coupling to the precursor peptide may optionally be converted to the free peptide boronic acid as described by Shenvi and Kettner in U.S. Patent 4,499,082 and J. Biol. Chem. 259:15106 (1984).

Where H-Y is an alphadiketone or alpha-keto ester (i.e., R3 is COCOCH₃ or COCOOC₂H₅) the requisite amino diketones or alpha-keto esters may be prepared as described by Angelastro et al., J. Med. Chem. 33:13 (1990) and references therein.

Where H-Y is a trifluoromethyl ketone (i.e., R3 is COCF₃) the requisite amino ketones may be prepared as described by Imperiali and Abeles, Biochemistry 25:3760 and supplementary pages (1986).

The amino-terminal substituents W and R in formulas II, III and V are derived from the corresponding carboxylic acids W-OH and R-OH which may be purchased commercially (e.g., from Narchem Corporation, Chicago, IL) or prepared as described in the following paragraphs using techniques familiar to those skilled in the art.

The aminoalkanecarboxylic acids (W-OH as required for formula V compounds) may be prepared by sequential alkylation of ethyl malonate by an alkyl or arylalkyl bromide followed by an omega-phthalimido-alpha-bromoalkane. The resulting substituted malonic ester may be hydrolyzed by mild base and decarboxylated by treatment with mild acid to yield an omega-phthalimido-alpha-alkyl (or arylalkyl)-substituted alkanecarboxylic acid. This intermediate may be chain extended by the Arndt Eistert homologation reaction (Eistert in "*Newer Methods in Preparative Organic Chemistry*", 1:513). Finally, strong base hydrolysis is used to remove the phthalimido protecting group to give W-OH.

The substituted alkane carboxylic acids (R-OH) as required in formulas I and II may similarly be prepared by the sequential alkylation of ethyl malonate with alkyl or arylalkyl bromides follow as above by mild basic hydrolysis and mild acid decarboxylation. The resulting alpha-substituted alkane carboxylic acid may be extended as above by the Arndt-Eistert homologation reaction.

The omega monoesters of substituted alkanecarboxylic acids (R-OH as required in certain cases in formulas I and II) may be prepared by the sequential alkylation of ethyl malonate with an alkyl or arylalkyl bromide followed by alkylation with the methyl or ethyl ester of an omega-bromo (or -chloro) alkane carboxylic acid. Followed by mild acid hydrolysis and decarboxylation the resulting alpha-substituted alkane dicarboxylic acid omega-ester may be extended as above by the Arndt-Eistert homologation reaction.

The resulting racemic carboxylic acids (W-OH and R-OH) may be resolved into their enantiomeric forms by passage through a chiral chromatographic column or by conversion to diastereoisomeric salts of asymmetric bases such as quinine, cinchonine, brucine and the like followed by fractional crystallization.

### Example 1: Preparation of Precursor Peptides

These compounds were prepared by solution phase methods using N-hydroxysuccinimide esters (Anderson et al., J. Am. Chem. Soc. 86:1839, 1964) or mixed carbonic anhydrides (Anderson et al., J. Am. Chem. Soc. 89:5012, 1967).

The N-hydroxysuccinimide esters were prepared by treatment of a solution of 2 grams of the protected amino acid or peptide in 25 ml of dioxane with a molecular equivalent of N-hydroxysuccinimide and a molecular equivalent of diisopropylcarbodiimide (DIPCDI). The solution was stirred overnight at room temperature, and solvent was then evaporated under reduced pressure. The residue was dissolved in ethyl acetate, washed with water, 5% aqueous citric acid, and again with water. The mixture was dried (MgSO₄), filtered, and the solvent removed under reduced pressure on a rotary evaporator. The purity of the resulting solid product was verified by thin layer chromatography. From 2.0g of Fmoc-Val, 0.75g of N-hydroxysuccinimide and 0.70 ml of DIPCDI was obtained 2.6g of Fmoc-Val-N-hydroxysuccinimide ester (Fmoc-Val-Osu). 6.5g of succinic acid monomethyl ester and 6.1g of N-hydroxysuccinimide and 6.5 ml of DIPCDI in 60 ml of dioxane yielded 8.4g of Methoxysuccinyl N-hydroxysuccinimide ester (MeOSuc-Osu). In the last case it was found that the water washing caused a significant loss of product, thus minimum quantities of water were employed.

These N-hydroxysuccinimide eaters belong to a group of compounds called active esters which react readily with the primary or secondary amino groups of amino acids in aqueous or partially aqueous solution to yield peptides in high yield as follows:

### a) MeOSuc-Glu(t-Bu)

A solution of 1.0g glutamic acid-gamma-t-butyl ester in 5 ml water and 0.4 ml triethylamine was treated with a solution of 1.1g MeOSuc-Osu in 10 ml dioxane. The solution was stirred for 3 hours and the organic solvents were removed under reduced pressure on a rotary evaporator. The residue was taken up in 50 ml ethyl acetate and the resulting solution was washed once with water, three times with 5% aqueous citric acid, and finally with water. The organic layer was dried (MgSO₄), and the solvent was removed under reduced pressure. The residual, pale yellow oil, was dissolved in ether and 400 µls dicyclohexylamine was added. The resulting crystalline precipitate was filtered off and washed with small portions of ether and petroleum ether. The yield of N-Methoxysuccinyl glutamic acid gamma-t-butyl ester (MeOSuc-Glu (t-Bu)) was 0.9 gram.

### b) Fmoc-Val-Lys (Boc)

A suspension of 1.0g epsilon-t-butyloxycarbonyl lysine in 10 ml water and 0.40 ml N-methylmorpholine (NMM) was treated with a solution of 1.3g Fmoc-Val-Osu in 10 ml dioxane. The initially cloudy suspension became clear after stirring overnight. The resulting solution was treated with 20 ml 2% aqueous sodium bicarbonate solution and was twice extracted with ether. The ether extracts were discarded, and the aqueous solution acidified with dilute hydrochloric acid to pH 1.5 and extracted with three 75 ml portions of ethyl acetate. The combined extracts were washed once with water and dried (MgSO4), and solvent was removed under reduced pressure to yield 1.2g solid Fmoc-Val-Lys(Boc).

### c) MeOSuc-Glu(t-Bu)-Val-OBzl

A solution of 11.6g Fmoc-Glu(t-Bu)-Val-OBzl in 50 ml methylene chloride was treated with 20 ml piperidine and stirred for 30 minutes at room temperature. Solvent was removed under reduced pressure and remaining traces of piperidine removed by the repeated addition and evaporation of ethyl acetate portions. The residual gamma-t-butyl-glutamyl valine benzyl ester was a pale yellow solid. It was dissolved in 30 ml of a 1:1 mixture of dimethylformamide (DMF) and dimethyl sulfoxide (DMSO) and mixed with a solution of the monomethyl succinate acid N-hydroxysuccinimide ester obtained from 40 millimoles (5.28g) of methoxysuccinic acid. To this solution 2.6 ml NMM was added, and the reaction mixture stirred at room temperature overnight. Ethyl acetate (250 ml) was added, and the resulting solution extracted once with 100 ml water, three times with 100 ml 3% citric acid, three times with 100 ml 2% sodium bicarbonate and finally with 100 ml water. The organic layer was dried (MgSO₄) and evaporated to yield 7.8 grams of pale yellow solid N-methoxysuccinyl gamma-t-butylglutamyl valine benzyl ester.

### d) MeOSuc-Glu(t-Bu)-Val-Osu

A solution of 7.6g MeOSuc-Glu(t-Bu)-Val-OBzl in 60 ml acetic acetic acid was mixed with 2g 10% palladium-on-carbon in 50% water and hydrogenated at 40 p.s.i. for 3.5 hours. The product was filtered, and solvent evaporated to give 7.1g of sticky solid methoxysuccinyl gamma-t-butyl-glutamyl-valine. This product was dissolved in 60 ml tetrahydrofuran (THF), and 2.3g N-hydroxysuccinimide and 2.6 ml DIPCDI added. The reaction mixture was stirred overnight and solvent then evaporated. The residue was stirred with 200 ml ethyl acetate and the precipitated diisopropyl urea filtered off and discarded. The filtrate was washed with 100 ml water, 2% sodium bicarbonate (3x100ml) and 100 ml water, dried over MgSO₄, and then evaporated. The resulting solid methoxysuccinyl t-butyl glutamyl valine N-hydroxysuccinimide ester weighed 8.2 grams.

### e) MeOSuc-Glu(t-Bu)-Val-Lys(Boc)

The MeOSuc-Glu(t-Bu)-Val-Osu (8.2g) prepared above was dissolved in 70 ml THF and added to a solution of 4.9g epsilon-t-butyloxycarbonyl lysine in 60 ml water and 2.6 ml triethylamine. After stirring overnight the reaction mixture was diluted with 100 ml 2% sodium bicarbonate solution and extracted with ether. The aqueous solution was acidified with 3% citric acid and extracted with four 100 ml portion of ethyl acetate. The combined organic layers were washed with 100 ml water, dried (MgSO₄) and evaporated to give 7.0 grams of hygroscopic solid methoxysuccinyl-gamma-t-butyloxycarbonyl lysine.

### f) Fmoc-Glu(t-Bu)-Val-OBzl

This compound, as well as several in the next example, was prepared by a modification of the method of Anderson et al., J. Am. Chem. Soc. 89:5012 (1967).

A solution of 8.5 grams (20 millimoles) N-fluorenylmethoxy-carbonyl-gamma-t-butylglutamic acid in 80 ml anhydrous DMF was treated with 2.2 ml of NMM and cooled to -20°C under dry argon. A total of 2.6 ml isobutyl chloroformate was then added dropwise. The solution was stirred for 10 minutes and a precooled (-20°C) mixture of 6.25g (25 millimoles) valine benzyl ester hydrochloride and 3.3 ml NMM in 50 ml DMF was added slowly. The reaction mixture was stirred overnight and then dissolved in 300 ml ethyl acetate, and washed successively with water (1x100ml), 3% citric acid (3x100ml), dried (MgSO4) and evaporated. The residue was dissolved in ethyl acetate and diluted with ether to give 11.8g of fluffy white crystalline fluorenylmethoxycarbonyl-gamma-t-butyl-glutamyl-valine benzyl ester.

### Example 2: Preparation of Peptide Substrates

### a) Fmoc-Val-Lys(Boc)-Met-pNa

A solution of 1.1 grams of Fmoc-Val-Lys(Boc), 0.8 grams of BOP and 1.3 grams of 1-hydroxybenzotriazole (HOBt) in 10 ml DMF was stirred for 10 minutes. A solution of 0.6g methionine p-nitroanilide and 1.5 ml acetic acid in 5 ml DMF and 0.3 ml NMM was then added. The reaction mixture was stirred overnight and diluted with 75 ml ethyl acetate. The solution was washed with water (2x30ml), 2% sodium bicarbonate (3x30ml), water (1x30ml), 3% citric acid (2x30ml), and water (1x30ml), and was dried (MgSO₄) and evaporated under reduced pressure. The residue was dissolved in ethyl acetate and ether was added to precipitate 1.5g of a pale yellow solid fluorenylmethoxycarbonyl-valyl-epsilon-t-butyloxycarbonyl-ly syl-methionine-p-nitroanilide.

### b) Val-Lys(Boc)-Met-pNa

A solution of 1.5g Fmoc-Val-Lys(Boc)-Met-pNa in 20 ml methylene chloride was treated with 10 ml pyrrolidine. After 20 minutes the solvent was evaporated under reduced pressure. Remaining traces of pyrrolidine were removed by repeated dilution with ethyl acetate followed by evaporation under reduced pressure. The resulting solid: valyl-epsilon-t-butyloxycarbonyl-lysyl-methionine-p-nitroani lide weighed 1.35g after washing with petroleum ether.

### c) MeOSuc-Glu-Val-Lys-Met-pNa

A solution of 0.8g of MeOSuc-Glu(t-Bu) dicyclohexylamine salt, 1.3g of Val-Lys(Boc)-Met-pNa, 200mg of HOBt, 0.66g of BOP in 10 ml DMF was treated with 100 µl of NMM. The reaction mixture was stirred for 3 days and then diluted with ethyl acetate and washed with water, citric acid, and sodium bicarbonate and water as above. The organic layer was dried (MgSO₄) and evaporated to give a pale yellow solid which was purified by elution through a column prepared from 85g silica gel in chloroform. The first fraction, which was eluted with 150 ml chloroform, was discarded. The product was next eluted with 300 ml of a solution of 2% methanol in chloroform. The resulting eluate was evaporated to give a colorless solid (0.45g) which was dissolved in 10 ml methylene chloride and 10 ml TFA. After 20 minutes the solvents were evaporated under reduced pressure to give 270 mg of fluffy white solid methoxysuccinyl-glutamyl-valyl-lysyl-methionine-p-nitroanili de, which was purified by high pressure liquid chromatography (HPLC) in water using a gradient of 10 to 80% acetonitrile containing 0.1% TFA. The pure product weighed 80 milligrams.

### d) MeOSuc-Glu-Val-Lys-Phe-pNa

This compound and the following two were prepared by a mixed carbonic anhydride procedure. A solution of 1.93g (3.0 millimoles) of MeOSuc-Glu(t-Bu)-Val-Lys(Boc) in 10 ml DMF and 0.33 ml NMM was cooled to -20°C, and 0.39 ml isobutyl chloroformate was added. The reaction mixture was stirred for 10 minutes at -20oC, and divided into three portions for this and the following two sections.

A 40% portion of the reaction solution containing 1.2 millimoles of the mixed isobutyl carbonic anhydride of MeOSuc-Glu(t-Bu)-Val-Lys(Boc) was added under argon to a solution of 285 mg phenylalanine-p-nitroanilide (Bachem Biosciences, Philadelphia, PA.) in 5 ml DMF cooled to -15oC. After 30 minutes the reaction mixture was transferred to ice water and allowed to warm to room temperature over 2.5 hours. The reaction mixture was then diluted with 20 ml ether to precipitate a solid, which was filtered off, washed with ether and with petroleum ether, and dried. This solid was dissolved in 10 ml of a 1:1 mixture of methylene chloride and TFA. After 15 minutes, solvent was evaporated under reduced pressure, and the residue was triturated with ether to yield crude solid methoxysuccinyl-glutamyl-valyl-lysyl-phenylalanine-p-nitroan ilide. This was purified by HPLC in water containing 0.1% TFA using a 40 minute gradient of 25 to 90% acetonitrile containing 0.1% TFA.

### e) MeOSuc-Glu-Val-Lys-D-Phe-pNa

This compound was made by the procedure given in section (d) above, except that D-Phenylalanine-nitroanilide was substituted for L-phenylalanine-p-nitroanilide.

### f) MeOSuc-Glu-Val-Lys-Leu-pNa

This compound was made by the procedure given in section (d) above, except that leucine-p-nitroanilide was substituted for phenylalanine-p-nitroanilide.

The other protease substrates depicted in Fig. 11 were made by analogous methods.

### Example 3: Preparation of Peptide Inhibitors

These compounds were prepared by the mixed carbonic anhydride procedure described in the previous example. In general, a completely protected precursor peptide carboxylate is converted to a mixed isobutylcarbonic anhydride and allowed to react at low temperature with the free amino group of the A1 residue which may optionally have its free functional groups protected as esters, ethers or acetals.

### a) MeOSuc-Glu-Val-Lys-Phenylalanine chloromethylketone [N-Methoxysuccinyl-glutamyl-valyl-lysyl-(1-chloro-4-phenyl-2 -keto-3-butylamide)].

The mixed isobutyl carbonic anhydride of methoxysuccinyl-gamma-t-butyl-glutamyl-valyl-epsilon-t-butox ycarbonyl lysine was prepared by dissolving 1.93g (3.0 millimoles) of this protected precursor peptide in 10 ml DMF and 0.33 ml NMM. The solution under dry argon was cooled to -20oC and 0.39 ml isobutyl chloroformate added. This reaction mixture was stirred for 10 minutes at -20°C, and divided into portions for this and the next two sections.

A 20% portion of the reaction solution (containing 0.6 millimoles of the precursor peptide mixed carbonic anhydride) was withdrawn and added to a solution of 142 mg (0.5 millimoles) of phenylalanine chloromethyl ketone hydrochloride (Bachem Biosciences, Philadelphia, PA.). The resulting solution was allowed to stir for three hours at room temperature, and then diluted with ether and filtered.

The filtrate was diluted with petroleum ether to precipitate MeOSuc-Glu-(t-Bu)-Val-Lys(Boc)-phenylalanine chloromethyl ketone which was collected by filtration. This product was added to 10 ml of a 1:1 mixture of methylene chloride and TFA. After 15 minutes the solvents were evaporated under reduced pressure. The residue was triturated with ether to give solid MeOSuc-Glu-Val-Lys-Phenylalanine chloromethylketone. This was purified by HPLC in 0.1% aqueous TFA on a C18 column with a 40 minute gradient of 25 to 90% acetonitrile.

### b) MeOSuc-Glu-Val-Lys-Borophenylalanine pinacol ester

A 20% portion of the mixed carbonic anhydride of MeOSuc-Glu(t-Bu)-Val-Lys(Boc) prepared in section (a) above was treated with 142 mg borophenylalanine pinacol ester (Shenvi, U.S. Patent 4,537,773; Shenvi and Kettner, J. Biol. Chem. 259, 15106,1984), in 2 ml DMF chilled to -15°C. The solution was stirred for 30 minutes at -15°C, then placed in ice and allowed to warm to room temperature over 2.5 hours. The reaction mixture was diluted with ether and filtered. The filtrate was evaporated, and the residue dissolved in chloroform and loaded on a silica gel column (10g; E. Merck Keiselgel 100). The product was eluted with 1:1 chloroform:methanol and fractions were monitored by thin layer chromatography (TLC).

The eluate was evaporated and the residue dissolved in 10 ml of 1:1 methylene chloride:TFA to remove the protecting groups. After 15 minutes solvent was evaporated and the residue triturated with ether. The resulting solid peptide product was purified by HPLC using conditions similar to those employed in section (a).

### (c) MeOSuc-Glu-Val-Lys-Boronorleucine pinacol ester.

A 20% portion of the mixed carbonic anhydride of MeOSuc-Glu(t-Bu)-Val-Lys(Boc) prepared in section (a) above was treated with 83 mg boronorleucine pinacolester (prepared by the method described in Shenvi and Kettner, supra), in 2 ml DMF. The reaction, workup and purification were carried out as described for the boro-Phe analog in (b) above.

### (d) MeOSuc-Glu-Val-Lys-Norleucinal

A solution of 2.0 millimoles mixed carbonic anhydride was prepared from 1.29g MeOSuc-Glu(t-Bu)-Val-Lys(Boc), 7 ml DMF, 0.22 ml NMM and 0.25 ml isobutyl chloroformate by the procedure described in section (a) above.

The resulting solution was added to a solution of 500 mg norleucinal di-n-butyl acetal (Gacek et al. Tetrahedron 30:4233, 1974) in 3 ml DMF. The reaction was carried out and worked up as described in section (b) above, and the resulting product purified by HPLC.

### (e) MeOSuc-Glu-Val-Lys-Phenylalaninal

This compound was prepared by the procedure of Example 3D except that phenylalaninal diethyl acetal was substituted for the norleucinal derivative, and a one hour period of deprotection with 1:1 methylene chloride/TFA was employed. The product was purified by HPLC and the structure was verified by fast-atom bombardment mass spectroscopy (FAB-MS). Called M+1 = 620. Found M+1 = 620.

### (f) MeOSuc-Glu-Val-Lys-Aza-Phe-NH₂

Potassium cyanate (0.73g, 0.009 mole) was added in small portions over 30 minutes to a solution of 1.00g (0.0045 mole) of t-butyl 3-benzylcarbazate, and 4.5 ml of 4N hydrochloric acid in 5 ml of water and 5 ml of dioxane. The mixture was stirred overnight, and the white crystalline product was filtered off and washed with aqueous 20% citric acid, water, and after vacuum drying weighed 0.81g This solid was decrystallized from ethyl acetate-hexane to give 0.63g of N-t-Butoxycarbonyl-alpha-azaphenylalanine amide (Boc-AzaPhe-NH₂), m.p. 131-131.5°C (Lit. m.p. 124-125°C, Dutta et al., *J. Chem. Soc. Perkin I* (1975) 1712)

A solution of 0.6g of the above amide in 5 ml of dioxane and 5 ml of 4N HCl in dioxane was allowed to stand 1 hr, and the solvent was evaporated and 20 ml of ether was added. The precipitate (0.52g) was collected, washed with ether and dried. A 0.28g portion of the solid in 3 ml of DMF was treated with 1.2g of MeOSuc-Glu(t-Bu)-Val-Lys(Boc) (described in Example 1(e) above) and 0.33g of BOP and 0.11g of HOB_{T} was added. The pH was adjusted by the dropwise addition of NMM (ca. 0.2ml) using a droplet of the reaction solution applied to moist pH paper to give a pH measurement of 7.5. The solution was allowed to stand for 2 days and was diluted with 75 ml of ethyl acetate, and was worked up by the procedure in Example 1(c) above. The organic layer was evaporated to give 0.25g of a gummy solid which was taken up in 10 ml of 1:1 CH₂Cl₂/TFA. After 1 hr, 25 ml of ether was added to precipitate 0.15g of MeOSuc-Glu-Val-Lys-Aza-Phe-NH₂. The product was purified by HPLC and the identity was verified by FAB-MS.

### Proteases

Proteases of this invention are chymotrypsin-like serine proteases derived from mammalian tissues. These proteases share an unusual substrate specificity in being able to cleave a Met-Asp bond in a beta-amyloid precursor protein. This aberrant cleavage is required to liberate beta-amyloid in Alzheimer's disease. The encompassed proteases have an enzymatic activity, as described above, which is similar to, or identical to, that activity naturally occurring in such mammals. These proteases may be isolated and purified, as generally described above, and used as purified preparations. Alternatively, the amino acid sequence of the isolated protease, or tryptic or other fragments thereof, can be determined by standard techniques and the nucleic acid encoding that protease isolated. The active site of the protease can be similarly determined by fragmenting the enzyme and testing its activity, e.g., as described below. Those fragments with the desired activity are useful in this invention. Fragments of the enzyme, like the intact enzyme, can also be created by standard recombinant DNA technology. Antibodies, e.g., monoclonal antibodies, to these proteases or fragments thereof are created by standard technology.

Purified protease can be used for the rational design of organic molecules which fit into the enzyme active site and inhibit its activity. The achievement of X-ray crystallographic structure of purified protease at high resolution will considerably aid this endeavor. Purified enzyme can also be used for large-scale screening of libraries of organic molecules to find compounds which inhibit protease activity.

Examples of isolation of two such proteases, chymase and the multicatalytic protease, are described below. The examples are not limiting to the invention. Those of ordinary skill in the art can readily isolate other proteases having such enzymatic activity using the protocol provided below, or equivalent protocols. Similarly, other proteases can be isolated by standard recombinant DNA technology using the amino acid sequence of chymase or the multicatalytic protease to allow reverse engineering of nucleic acid encoding chymase, the multicatalytic protease, or related enzymes.

During the various protease purification steps, protease activity of all fractions was measured using chromogenic or fluorogenic peptide analogs as substrates. Other methods are available, known to those skilled in the art. One specific example of an activity measurement is described here. Protease activity assays were performed in 96 well microtiter plates, in a total volume of 0.2 mls. Substrates were prepared as 10-20X concentrated stock solutions in DMSO. Therefore, each reaction contained 5-10% DMSO. Reactions were initiated by addition of protease-containing samples to wells already containing substrates, and were maintained at 37°C. Control wells received either protease without substrate, or substrate without protease. The release of reporter moities by proteolytic cleavage was followed as a function of time by either spectrophotometry (e.g., for pNa substrates, change in absorbance with time at 405 nm) or fluorimetry (e.g., for AMC substrates, change in fluorescence with time using excitation at 390 nm and emission at 460 nm). Maximum velocity of reactions was determined while substrate hydrolysis increased linearly as a function of time.

### Example 4: Chymase purification

Chymase (also known as mast cell protease I) was purified to homogeneity from rat brain generally as follows. Upon tissue homogenization and centrifugation, the enzyme was present in the particulate fraction. Chymase was enriched by removal of detergent-soluble proteins. Chymase was then solubilized from the particulate fraction using high salt, such as 1M MgCl₂ or 2M NaCl. Next, it was precipitated by dialysis into a low ionic strength buffer, and was collected by centrifugation. The pellet was suspended in buffer and fractionated by chromatography on a heparin-Sepharose column. Finally, chymase was purified to homogeneity by affinity chromatography using a substrate linked to resin. Purification steps were monitored by hydrolysis of the general chymotrypsin-like protease substrate Suc-Ala-Ala-Pro-Phe-pNa (Nakajima et al., J. Biol. Chem. 254:4027, 1979), and the APP substrate mimics MeOSuc-Glu-Val-Lys-Met-pNa and MeOSuc-Glu-Val-Lys-Phe-pNa. All procedures were performed at 4°C unless otherwise indicated.

### a) tissue extractions

Forebrains from 5-7 day old rat pups (17g) were homogenized using a glass/Teflon motorized homogenizer in 295 mls 20 mM sodium phosphate (pH 7.5), 1 mM EDTA, and 1% Triton X-100. After 30 minutes with occasional stirring, this homogenate was centrifuged for 1 hr. at 40,000 x g in an SS34 rotor. The pellets were resuspended in 40 ml 20 mM sodium phosphate (pH 7.5), 1.2M MgCl2, and 0.1% Triton X-100 using a large Dounce homogenizer. To obtain a tight pellet during the next centrifugation, the viscosity of the mixture was reduced by two passages through a 20-gauge needle to shear nucleic acids. After 40 minutes with occasional stirring, the mixture was sonicated, then centrifuged as above for 1 hr. and the supernatant retained. This supernatant was then extensively dialyzed against a low ionic strength buffer (5 mM sodium phosphate, pH 7.5). The following day, particulates present in the dialysis bag were first resuspended by end-to-end mixing, then the dialysate was centrifuged as above for 1 hr. The resulting pellet was dissolved in a total of 8 ml 20 mM sodium phosphate (pH 7.5), 1 mM EDTA, 0.1% Triton X-100 ("buffer A"), plus 0.4 M NaCl and was used directly for the heparin agarose column.

### b) heparin affinity chromatography

The resuspension was sonicated, then applied in 1 ml aliquots to a column (1.5 x 2 cm) of heparin-agarose (Bio-Rad, Burlingame, CA.) equilibrated with buffer A plus 0.4 M NaCl. The column was washed with the same buffer until the concentration of eluting protein fell below 0.1 mg/ml. Proteins were then eluted from the column using a step-wise gradient of increasing NaCl concentration in buffer A. The protease showed an avidity for heparin, and was eluted with 0.9-1.2 M NaCl, with an approximate enrichment of 20-fold. The above methods for partial purification of chymase (mast cell protease I) have been described in the literature (e.g., Woodbury et al., Meth. Enzymol 80:588, 1981 and references therein).

### c) Substrate affinity chromatography

The concentrated, partially purified chymase-containing sample was loaded onto a column containing Sepharose resin to which chymase substrate analog, Ala-Pro-Phe, had been covalently conjugated. The free amino group of the substrate analog was linked to Sepharose that had been activated by cyanogen bromide. Chymase bound to the resin in the presence of high concentrations of NaCl or MgCl₂, e.g., 1M NaCl, and was eluted with 1M ammonium bicarbonate. Other methods are also available for chymase elution, e.g., reduction of NaCl to 0.5 M in 40% ethylene glycol. Eluted fractions were monitored for chymase activity as below.

### d) Analyses of purified chymase

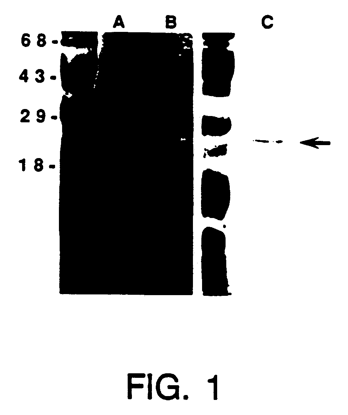

The eluate from the final column contained a closely-spaced doublet of ∼25-26 kDa detectable by SDS-polyacrylamide gel electrophoresis with ultrasensitive silver staining (Nelson et al., J. Neurochem. 53:641, 1989; Merril et al., Anal. Biochem. 110:201, 1981). An illustration is provided in Fig. 1. The silver-stained chymase doublet is shown in lane C. These polypeptides were confirmed as proteases by enzymography. In this technique (Miskin et al., Anal. Biochem. 118:252, 1981; Nelson and Siman, J. Neurochem., supra), protein samples that contain proteases are fractionated by SDS-polyacrylamide gel electrophoresis in which a protease substrate (e.g., gelatin) is covalently incorporated into the gel matrix. Following electrophoresis, the SDS is removed and gels are incubated at elevated temperature (e.g., 37°C) to activate any proteases present. At locations within the gel containing active protease, the substrate will be cleaved from the gel matrix. The uncleaved substrate remaining within the gel is stained by a protein-binding dye, such as Coomassie blue or Amido black. With this technique, proteases appear as light areas (where substrate has been liberated from the gel matrix) against a darkly stained background. Purified chymase was examined by enzymography, shown in Figure 1 (Lane A is the heparin eluate, lane B the purified chymase; the left lane shows molecular weight standards, in kDa X 10-3). The two closely-spaced polypeptides in the purified chymase preparation (lane C) both exhibit protease activity (lane B). The chymase activity exactly co-migrated with the protein detected by silver staining.

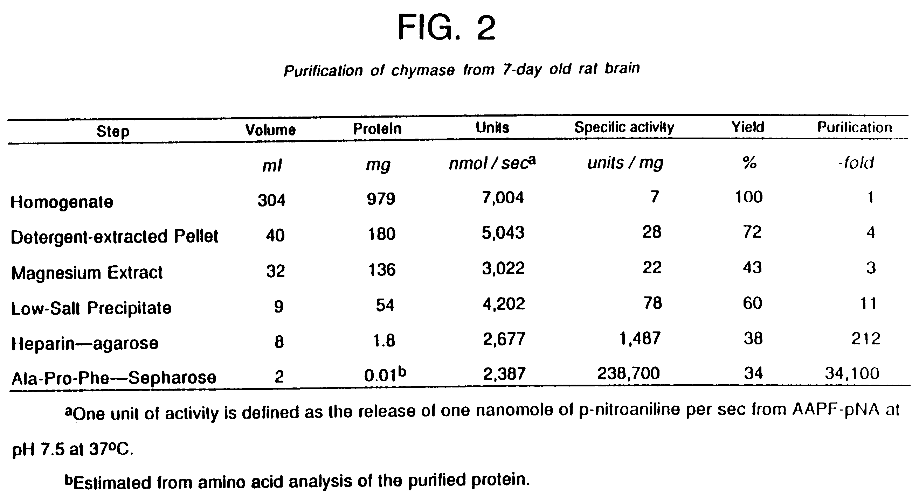

The purification scheme is summarized in Figure 2. Starting from 18g rat brain, approximately 10µg homogeneous chymase was obtained. The purified protease exhibited characteristics of the chymotrypsin-like protease chymase: (1) molecular weight of ∼25-26 kDa; (2) hydrolysis of peptide analog substrates with aromatic or large aliphatic amino acids at their carboxyl terminus (e.g., Suc-Ala-Ala-Pro-Phe-pNa, MeOSuc-Ala-Ala-Pro-Met-pNa); (3) irreversible inhibition by and formation of an SDS-stable complex with alpha1-antichymotrypsin.

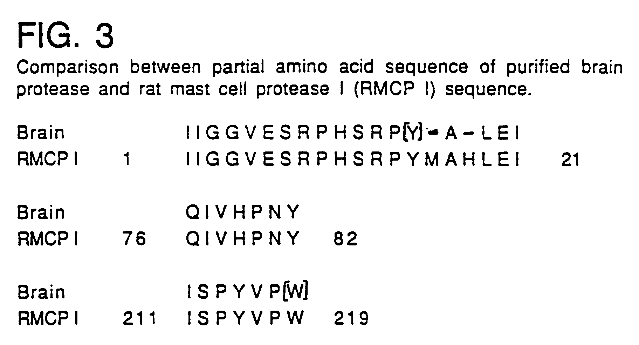

Amino acid sequencing was performed on purified brain chymase to confirm the identity of the protease. This procedure was carried out by the Harvard Microchemistry Facility using a method modified from Aebersold et al., Proc. Natl. Acad. Sci. 84:6970 (1987). Chymase that had been eluted from the final substrate affinity column with ammonium bicarbonate was lyophilized. Next, ∼150 pmol of chymase was digested with trypsin (enzyme:substrate of 1:20), and the resulting chymase fragments were separated by narrow-bore reverse phase HPLC. Three fragments were chosen for automated N-terminal microsequencing on the basis of their yields and predicted lengths, and sequenced in a gas-phase sequenator. Figure 3 compares the partial amino acid sequences obtained from three fragments of the purified protease with those published for authentic chymase (LeTrong et al., Biochemistry 26, 6988-6994, 1987). All 32 residues from the three domains precisely match the published sequence for chymase.

### Example 5: Multicatalytic protease purification from human brain

The multicatalytic protease identified in mammalian brain (Wilk and Orlowski, J. Neurochem. 40:842, 1983) has also been referred to in the literature as "ingensin" (Kamakura et al., J. Neursci. Res. 20:473, 1988), "lens neutral endopeptidase" (Ray and Harris, Biochem. J. 248:643, 1987), "proteasome" (Tanaka et al., J. Biol. Chem. 261:15197, 1986); J. Biol. Chem. 263:16209, 1988), or "macropain" (Rivett, Arch. Biochem. Biophys. 268:1, 1989). We identified and purified from human brain an enzyme that shares a number of features with the multicatalytic protease. The activity was present in the soluble fraction of a human brain homogenate, and bound to anion exchange and hydroxyapatite columns. Molecular weight of the protease was found to be extremely large (>600,000) by gel filtration, a step that afforded significant purification of the enzyme. Protease activity at each stage of the purification was measured as described above. A specific example of multicatalytic protease purification from human brain is described below.

### a) Tissue extraction and ion exchange chromatography

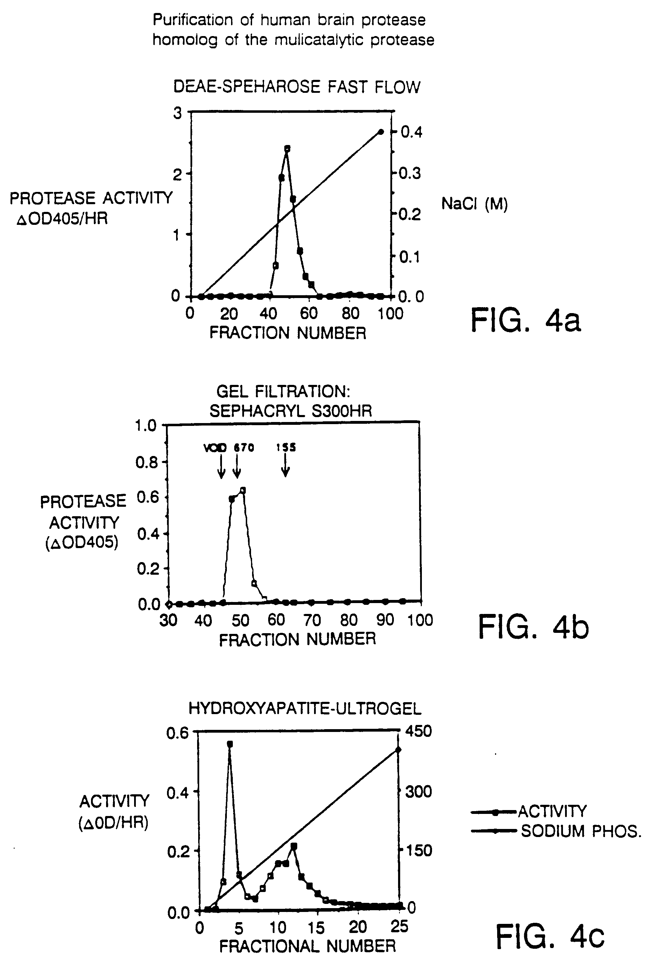

Human cerebral cortex (courtesy National Neurological Research Bank, Los Angeles, CA.) was obtained at autopsy and stored at -70°C until use. All procedures were conducted at 4oC unless noted otherwise. Three samples (35g) were pooled and homogenized in a Waring blendor in 200 mls 10 mM sodium phosphate (pH 7.4), 0.5 mM EDTA. Triton X-100 was added to 0.5%, the homogenate was stirred for 1 hr., then centrifuged at 40,000 x g for 1 hr. The supernatant, containing the multicatalytic protease, was dialyzed against 20 mM Tris-HCl (pH 8.0),1% glycerol. The following day, the dialysate was loaded on a column (150 ml bed volume) of DEAE-Sepharose Fast Flow (Pharmacia, Piscataway, N.J.) equilibrated in the dialysis buffer. Unbound material was removed with ∼300 mls dialysis buffer, then bound material was eluted with a linear NaCl gradient from 0 mM to 400 mM (1.5 liters total). Fractions of 15 ml were collected and assayed for multicatalytic protease activity (see below). Figure 4 (top) shows the elution profile of the protease from this column. Protease activity was detected in this and subsequent purification steps by hydrolysis of the APP-mimic substrate MeOSuc-Glu-Val-Lys-Met-pNa (see example 6 below for details) in the presence of 0.05% SDS, which is known to stimulate multicatalytic protease activity (Ishiura et al., supra).

### b) Gel filtration

Active fractions were pooled, brought to 30% saturated ammonium sulfate, stirred 30 minutes, and centrifuged at 40,000 x g for 30 minutes. The pellet was discarded, while the supernatant was brought to 85% saturation and stirred and centrifuged as before. The pellet, containing the multicatalytic protease, was suspended in 5 ml gel filtration running buffer (20 mM Tris-HCl, pH 7.5, 1% glycerol) and run on a 500 ml bed of Sephacryl S300HR (Pharmacia). The column was standardized with molecular weight markers blue dextran (>2,000 kDa), thyroglobulin (670 kDa), gamma-globulin (150 kDa) and ovalbumin (43 kDa). The multicatalytic protease eluted from the column with an apparent molecular weight of approximately 700 kDa (Figure 4, middle).

### c) Hydroxapatite chromatography

The human brain multicatalytic protease was further purified using hydroxyapatite-Ultrogel (IBF, Columbia, MD.); a 4 ml bed was equilibrated with 4 mM sodium phosphate (pH 7.4). Active fractions pooled from gel filtration were diluted with equilibration buffer and loaded onto the column at room temperature. Unbound proteins were washed off with several column volumes of equilibration buffer, then bound proteins were eluted with a linear gradient of sodium phosphate of 4 mM to 400 mM (60 mls). The multicatalytic protease eluted as illustrated in Figure 4 (bottom). This chromatographic step split the human brain protease activity into two peaks, as has previously been demonstrated for hydroxyapatite purification of the multicatalytic protease (Ishiura et al., supra).

Several features of the protease activity purified as above indicate that it is the human brain homolog of the multicatalytic protease: (1) size, ∼700 kDa; (2) co-purification; (3) chymotrypsin-like activity, hydrolyzing peptide analog substrates with aromatic or large aliphatic amino acids at the carboxy terminus; (4) marked stimulation of activity by low concentrations of SDS (Ishiura et al., supra); (5) inhibition by chymostatin, but not by several common protease blockers (e.g., (phenylmethylsulfonylfluoride, leupeptin, pepstatin, o-phenanthroline, EDTA).

### Example 6: Identification of proteases important to Alzheimer's disease.

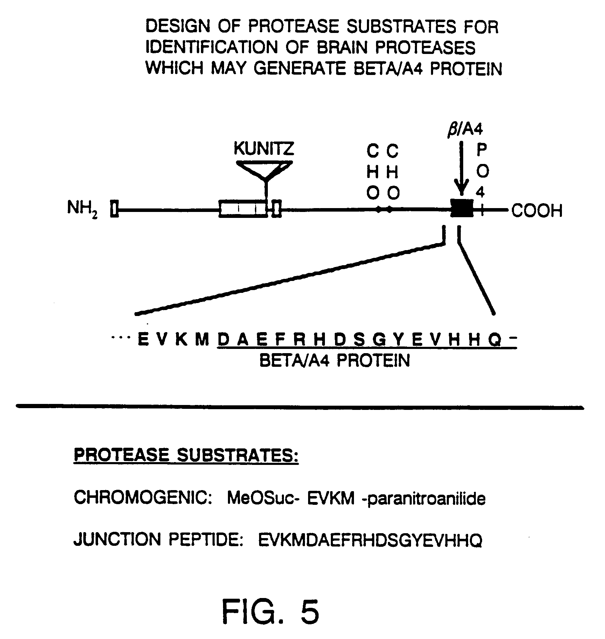

Neuritic plaques are generated in the brain in Alzheimer's disease by aberrant liberation of beta-amyloid from its precursor proteins (APPs). The sequence of beta-amyloid contained within APPs is shown in Figure 5. Cleavage of the bond between Met⁵⁹⁶ (M in the one letter amino acid code) and Asp⁵⁹⁷ (D) is required for beta-amyloid formation, and has been proposed to occur by action of a chymotrypsin-like protease (Abraham and Potter, Biotechnology 7, 147-153, 1989); Van Nostrand et al., Nature 341, 546-549, 1989). Figure 5 illustrates a strategy employed by the inventors for identifying chymotrypsin-like proteases which can liberate beta-amyloid in Alzheimer's disease. Two novel protease substrates were designed and synthesized: MeOSuc-Glu-Val-Lys-Met-pNa and the 19 amino acid peptide referred to as "junction peptide". Because cleavage between -Glu-Val-Lys-Met and Asp- liberates beta-amyloid, proteases cleaving these substrates on the carboxy terminal side of Met could be involved in Alzheimer's disease.

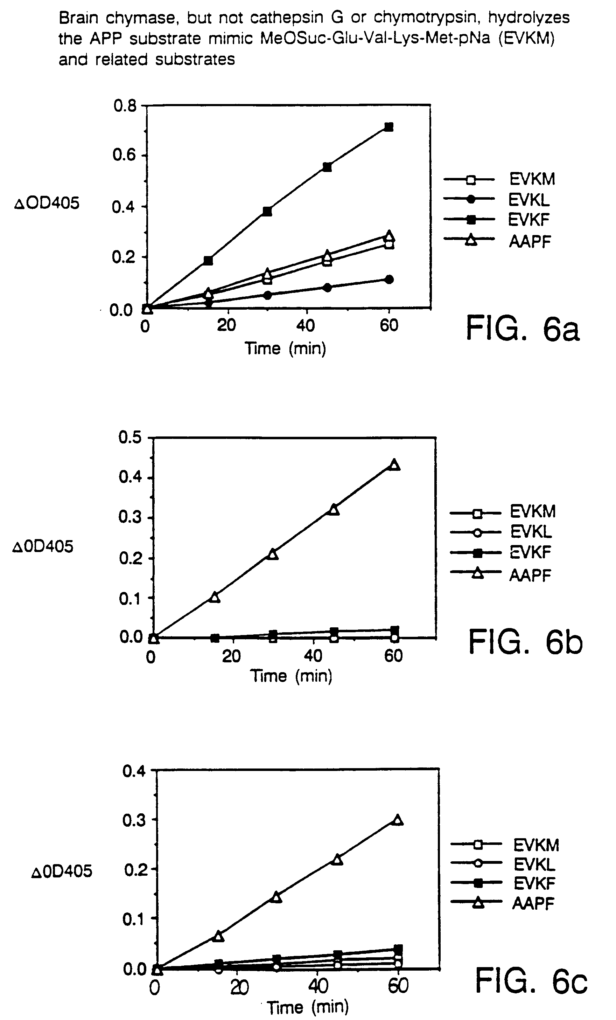

Contrary to expectation, not all chymotrypsin-like proteases were able to cleave MeOSuc-Glu-Val-Lys-Met-pNa (Figure 6; EVKM using the one letter amino acid code). Both chymase and the multicatalytic protease readily degraded this substrate, but the chymotrypsin-like enzymes cathepsin G and chymotrypsin were essentially without effect. This is particularly surprising, since both cathepsin G and chymotrypsin readily degraded another substrate with Met at the carboxyl terminus, MeOSuc-Ala-Ala-Pro-Met-pNa (e.g., Nakajima et al., supra). Selective cleavage of MeOSuc-Glu-Val-Lys-Met-pNa by chymase and the multicatalytic protease demonstrates that protease substrate specificity is markedly influenced by amino acid residues upstream of the cleavage site, and that not all chymotrypsin-like proteases are capable of liberating beta-amyloid. The results strongly implicate chymase and the multicatalytic protease in the abnormal production of neuritic plaque beta-amyloid.

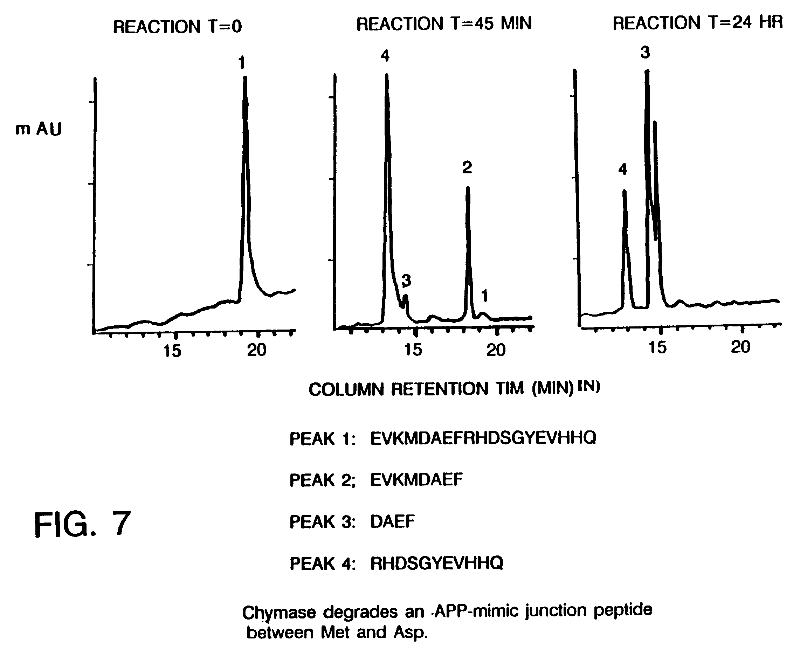

Chymase was also tested for cleavage of a long peptide whose sequence spans the junction of the amino terminus of the beta-amyloid domain (residue Asp⁵⁹⁷). The 19 amino acid junction peptide, Glu-Val-Lys-Met-Asp-Ala-Glu-Phe-Arg-His-Asp-Ser-Gly-Tyr-Glu-Val-His-His-Gln (corresponding to APP residues 593 to 611, numbering according to Kang et al., supra), was synthesized by solid phase methods and purified by reverse phase HPLC. The structure was confirmed by FAB-mass spectrometry and automated microsequencing. Purified chymase was incubated with purified junction peptide at an enzyme:substrate molar ratio of 1:50 at room temperature. The reaction mixture was fractionated by reverse phase HPLC immediately after adding enzyme, as well as at 45 and 90 minutes and 24 and 48 hrs. The intact junction peptide alone was run on the same HPLC column to identify its retention time. In as little as 45 minutes, intact junction peptide disappeared and was replaced by three prominent peptide fragments (Figure 7). Each of these fragments was identified by automated microsequencing. Based on the sequences of the junction peptide fragments, chymase degraded the junction peptide between Met and Asp. This further implicates chymase in the aberrant liberation of beta-amyloid in Alzheimer's disease.

The multicatalytic protease purified from human brain also exhibited an unusual ability to cleave the APP-mimic substrate MeOSuc-Glu-Val-Lys-Met-pNa (see example 8 below), like chymase but unlike the chymotrypsin-like enzymes chymotrypsin and cathepsin G. This implicates the multicatalytic protease in the aberrant liberation of beta-amyloid in Alzheimer's disease.

### Example 7: Human homolog of chymase

Human brain contains a homolog of rat chymase. The homolog was identified using the same two assays of chymase activity, hydrolysis of the chromogenic substrates Suc-Ala-Ala-Pro-Phe-pNa and MeOSuc-Glu-Val-Lys-Met-pNa, and enzymography, and was purified by a scheme similar to the one detailed above for the rat brain protease. The human brain chymase is similar to its rat counterpart in its co-purification, its inhibition by alpha1-antichymotrypsin, its hydrolysis of peptide analog substrates of chymase, and its apparent molecular weight of about 22,000 on nonreducing enzymographic gels. Chymase was also detected in mast cells in human Alzheimer's disease brain using antibodies to authentic human mast cell chymase (provided by N. Schechter, University of Pennsylvania, described in Schechter et al., supra). Specific examples of these methods are described below.

Samples obtained post-mortem from human cerebral cortex (courtesy of the National Neurological Research Bank) were fractionated as described above for preparation of rat brain chymase. The human brain activity exhibited detergent-insolubility, high salt solubility, and low salt insolubility. Protease was further purified on a heparin-Sepharose column in a manner similar to purification of rat brain chymase. Protease activity was monitored by hydrolysis of the substrate Suc-Ala-Ala-Pro-Phe-pNa as described above for rat chymase. The enzyme bound to the column in 0.4 M NaCl and was eluted by increasing the NaCl concentration to 0.7-0.9 M.

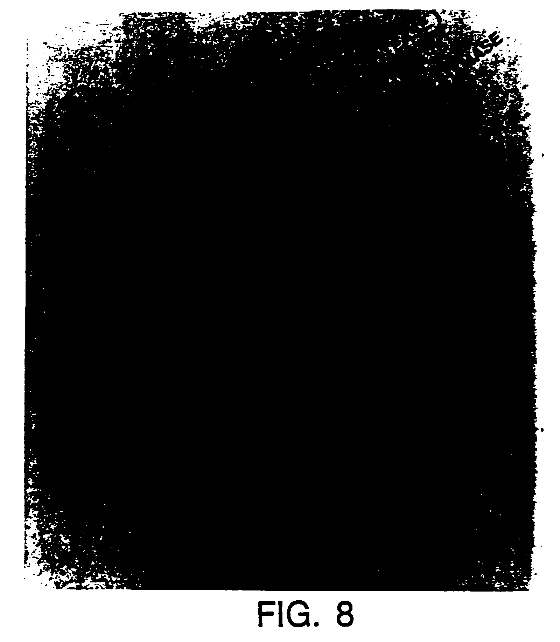

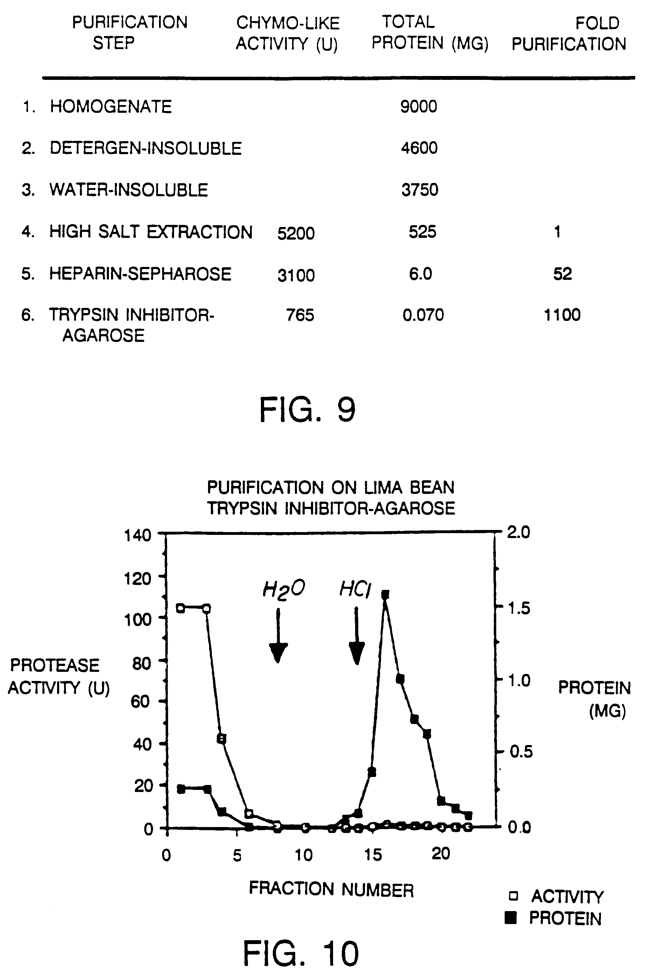

Next, chymase was purified using a chymase inhibitor coupled to resin, lima bean trypsin inhibitor-agarose (Sigma, St. Louis, MO.). This trypsin inhibitor binds to and blocks human mast cell chymase (Schechter et al., J. Biol. Chem. 258:2973, 1983). Chymase-containing fractions from the heparin eluate were pooled, concentrated to 10 ml, and loaded on a 1 ml bed volume of lima bean trypsin inhibitor-agarose. The column was washed with Hepes buffer containing 0.5 M NaCl (5 vol), then with water (5 vol). Chymase was eluted with 2 mM HCl, and was immediately neutralized with Tris-HCl (0.2 M final concentration, pH 8.0) containing 0.5 M NaCl. Figure 8 (right panel) summarizes the purification of the human brain protease. This enzyme hydrolyzed the general chymotrypsin substrate Suc-Ala-Ala-Pro-Phe-pNa and also the specific substrate MeOSuc-Glu-Val-Lys-Met-pNa. The enzyme activity was inhibited by alphal-antichymotrypsin. On enzymographic gels, as shown in Figure 8, the human brain chymase exhibited protease activity which co-migrated with rat brain chymase (purified as described above) and authentic human mast cell chymase (Schechter et al., 1983, supra). Figures 9 and 10 illustrate the results of the purification.

Mast cell chymase was also located in human brain by immunohistochemical analysis using an antiserum to chymase described above. In this procedure, thin sections of human brain (obtained post-mortem, fixed in aldehydes, and embedded in paraffin) are probed with rabbit antibody to human mast cell chymase. Bound rabbit anti-chymase is then detected by an indirect immunoperoxidase procedure (Sternberger, in Immunocytochemistry, Cohen et al. eds., pp. 104-169, Wiley, New York, 1979); Siman et al., Neuron 3:279, 1989). Using this procedure, chymase immunoreactivity was located within the secretory granules of mast cells in both control and Alzheimer's disease cerebral cortex.

### Example 8: Protease inhibitors

Inhibition of the proteases responsible for the aberrant liberation of beta-amyloid in Alzheimer's disease may slow the progression of the disease. Once the candidate proteases have been identified, on the basis of their ability to cleave the Met⁵⁹⁶-Asp⁵⁹⁷ bond in APP, the purified enzymes can be used to screen for protease inhibitors. Two examples of this approach are illustrated below, in the development of highly potent and selective inhibitors for the chymotrypsin-like proteases chymase and the multicatalytic protease.

To develop inhibitors for the two proteases, over 40 peptide analog substrates were prepared by the methods described in examples 1 and 2 above. Additional substrates were purchased from Sigma or Bachem. These substrates differ from one another by systematic, single residue substitutions; by comparing their cleavage by protease, the precise substrate preference of each protease was determined. In turn, this most-preferred sequence was used along with modifications of the carboxy terminal residue to generate highly specific inhibitors. Those skilled in the art will recognize that a number of carboxy terminal modifications are available which convert a serine protease substrate into an inhibitor (e.g., Powers et al., In Proteinase Inhibitors, Barrett et al. (eds) Elsevier, pp. 55-152, 1986). The inhibitors described below are not limiting to the invention; using equivalent approachs, nonpeptidic molecules can be identified from screening collections of organic molecules, or can be rationally designed based on knowledge of the enzyme active site obtained from the series of peptide substrates.

### a) Chymase inhibitors

Cleavage of 54 peptide analog p-nitroanilide substrates by brain chymase is depicted in Fig 11. At least 5 concentrations of each substrate were tested. Double-reciprocal plots were used to generate kinetic constants. In the figure, the Vmax/Km for each substrate is presented compared with substrate #1, MeOSuc-Glu-Val-Lys-Phe-pNa. Residues upstream of the cleaved bond are denoted by their "P" position (P1 is immediately upstream of the cleavage site, P2 is two residues upstream, etc.).

Note that compounds 1-10 compare distinct P1 amino acids. Among those examined, Phe was preferred by more than 8-fold over Nle, which in turn was preferred over Met and Leu. Other residues were not cleavable. Similar systematic comparisons were conducted for the P2, P3, and additional upstream sites. Of particular note is the chymase preferences at the P2 and P4 positions: a positively charged side chain is preferred at P2, while a negatively charged one is preferred at P4. This substrate specificity distinguishes chymase from other members of the chymotrypsin family. As already mentioned in example 6 above, neither chymotrypsin nor cathepsin G cleaved a substrate with negative charge at P4 (Glu) and positive charge at P2 (Lys), such as MeOSuc-Glu-Val-Lys-Met-pNa.

Based on the above chymase preferences, highly potent and selective inhibitors of the protease were prepared. By conversion of the carboxy terminal amino acid in a preferred substrate to an amino aldehyde, boronic acid, or chloromethylketone. Fig. 12 shows that the aldehyde, boronic acid, and chloromethylketone analogs of MeOSuc-Glu-Val-Lys-Phe-pNa each inhibited chymase with a Ki of less than 20 nM. These are substantially more potent than any chymase inhibitor previously described in the literature. Moreover, they are highly selective for chymase, as they inhibited other chymotrypsin-like proteases (Figure 12) or other proteases (Figure 11, panel A) only at much higher doses. For example, MeOSuc-Glu-Val-Lys-Phe-al, inhibits chymase with a Ki of 15 nM, but did not inhibit chymotrypsin or cathepsin G at doses up to 100 times greater (Figure 12). Furthermore, the inhibitor caused substantial blockade of the proteases trypsin, thrombin, elastase, urokinase, plasmin, or plasma kallikrein only at 10 uM concentrations or higher (Figure 11, panel A).

The aldehyde analogs of MeOSuc-Glu-Val-Lys-Phe and MeOSuc-Glu-Val-Lys-Nle were compared as chymase inhibitors. The Phe compound was approximately 20 times more potent than the Nle inhibitor, a finding consistent with the differential cleavage of the two p-nitroanilide substrate versions of these compounds.

### b) Multicatalytic protease inhibitors

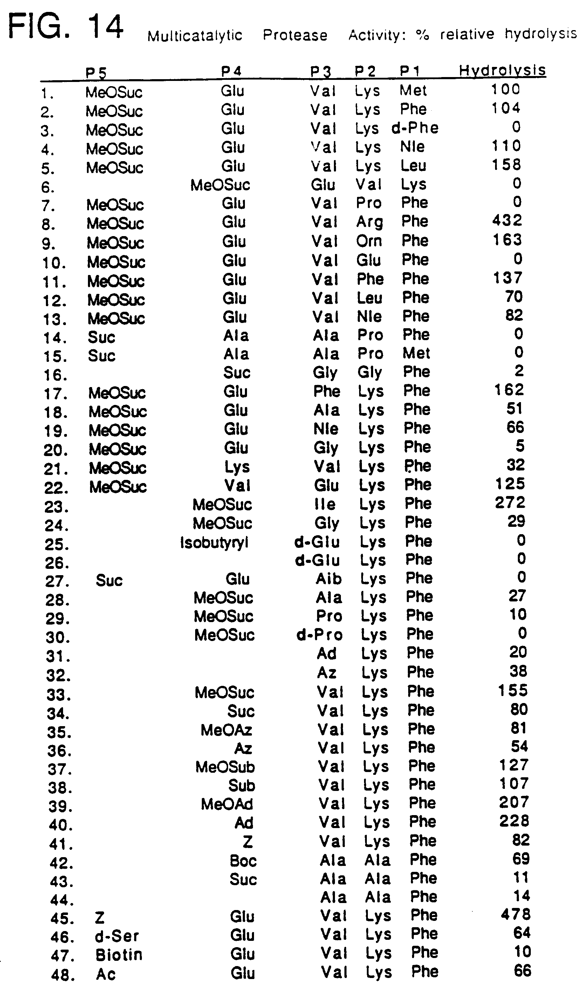

A strategy equivalent to that used to design chymase inhibitors was employed for the human brain multicatalytic protease. Figure 14 shows the relative hydrolysis rates of 48 peptide analog substrates, compared at a single substrate concentration. Note that substrate preferences differ from those of brain chymase. For example, while chymase prefers Phe at P1 and Lys at P2, the multicatalytic protease prefers Leu at P1 (although Phe, Nle, and Met are also excellent here) and Arg at P2. Among the residues tested thus far at the P3 position, Ile is the most-preferred. Thus, peptide analogs can be carefully tailored which target the multicatalytic protease but spare related chymotrypsin-like enzymes.

As illustrated above for chymase, preferred substrates of the multicatalytic protease can be modified at their carboxy termini to generate potent enzyme inhibitors. Figure 13, panel B depicts the inhibition of the multicatalytic protease by some peptide analogs and commercially available protease inhibitors. The novel peptide analog inhibitors are substantially more potent blockers of the multicatalytic protease chymotrypsin activity than any previously described inhibitor.

### Example 9: AD and protease activity

Chymotrypsin-like protease activity was quantified in a number of human brain samples, in order to determine if chymase might be responsible for the aberrant proteolysis in brain in AD. Ninety-five brain samples from four brain regions were obtained from the National Neurological Research Bank, Los Angeles, CA. Forty-seven were from patients diagnosed as having AD (diagnosis confirmed at time of death), and forty-eight were from non-demented age-matched individuals who died of various causes. Chymase was enriched and separated from other protease activities by its insolubility in detergent and low-salt buffer, followed by solubilization in 1 M MgCl₂ (high salt extract preparation, as previously detailed). Chymase activity was assessed by hydrolysis of the general chymotrypsin substrate MeOSuc-Ala-Ala-Pro-Phe-pNa and was normalized to total protein content.

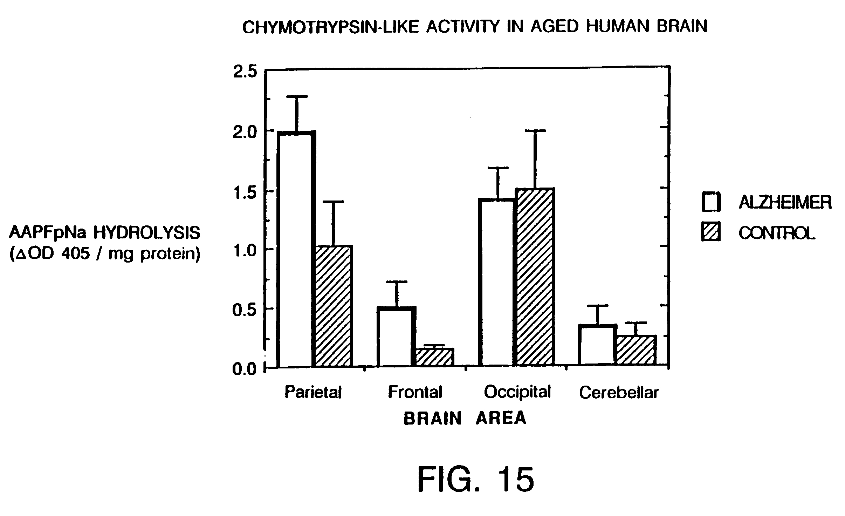

In AD parietal cortex, chymase activity is significantly elevated by about 2-fold (n= 17; p<0.05; Figure 15). Activity is also elevated in frontal cortex (n=24), but is unchanged in occipital cortex (n=26) and cerebellum (n=28). The differences in parietal and frontal cortex could not simply be ascribed to changes as a function of age or post-mortem sample autolysis time. Moreover, activity of two other proteases, cathepsin B and prolylendopeptidase, were not elevated in the same parietal cortex samples, indicating that there is no generalized increase in protease activities in AD. It is noteworthy that the two brain areas exhibiting elevated chymotrypsin-like activity are severely afflicted by AD, whereas the two areas exhibiting no elevation of protease activity are relatively spared by the disease (Brun et al., Histopathology 5 549, 1981); Kemper, in K. Nandy, ed., Senile Dementia: A Biomedical Approach, Elsevier, pp. 105-113, 1978). These results provides direct support for the idea that aberrant proteolytic activity of chymase might underlie the pathogenesis of AD.

### Therapy

The above inhibitors can be provided in any standard pharmaceutically acceptable buffer for administration to a patient suffering from AD or Down's Syndrome. Such inhibitors can be administered by standard procedures preferably at a level between 10-1000 µg/kg, intraveneously, intramuscularly, intranasally, orally or directly into brain tissue.

### Other Embodiments

### a) Nucleic acid encoding protease

Purified chymase or multicatalytic protease can be used for the cloning and sequencing of chymase and multicatalytic protease cDNA and protein, by methods now familiar to those skilled in the art of molecular biology of proteins. Partial amino acid sequence can be obtained from purified human brain chymase using automated or manual peptide sequencing employing Edman degradation or other chemistries. The partial protein sequence can be used in two ways. In one, antibodies are raised against chymase. A synthetic peptide is prepared based on the partial sequence, and is conjugated to a carrier protein and used to raise polyclonal and monoclonal antibodies by conventional methods (Harlow et al., Antibodies, A Laboratory Manual, Cold Spring Harbor Laboratory, New York, 1988). In the second, chymase cDNA is cloned and sequenced, thus providing the full nucleotide and amino acid sequences of the protein. An oligonucleotide is prepared based on the partial protein sequence, and is used to screen a cDNA library to select recombinant bacteria expressing chymase cDNA. Upon selection, the chymase cDNA is cloned and sequenced by conventional methods (Sambrook et al., Molecular Cloning, A Laboratory Manual, Cold Spring Harbor Laboratory, New York, 1989).

Alternatively, oligonucleotides based on the published amino acid sequence of rat chymase (LeTrong et al., Biochemistry 26:6988, 1987) can be used as primers for a polymerase chain reaction in the cloning of cDNA for rat or human chymase. Either the human cDNA can be directly obtained by using human mRNA to drive the reaction, or the rat cDNA can be obtained (by using rat mRNA to drive the reaction) and subsequently used as a probe for cloning the human counterpart by conventional methods.

Nucleic acid probes specific for chymase or the multicatalytic protease have utility in diagnostic formats using readily accessible cells. Mutations, duplication, or overexpression of the genes for clipsin or the multicatalytic protease can be detected by a variety of methods known to those skilled in the art.

### b) Antibodies to protease

Antibodies to chymase, the multicatalytic protease, or related proteases can be used in a variety of formats designed to detect protease aberrations which may be present in Alzheimer's disease. These have utility as diagnostic tests for the disease. For example, changes in the amount of chymase protein, or mutations in the chymase protein which lead to the production of altered forms, could be detectable in readily accessible cells such as skin fibroblasts or mast cells, or white blood cells. Antibody-based detection could be accomplished by a number of means presently in wide use by those skilled in the art, including Western blotting and enzyme-linked immunosorbant assay (ELISA; Harlow and Lane, Antibodies, A Laboratory Manual, Cold Spring Harbor Laboratory, New York, 1988).

### c) Protease inhibitors

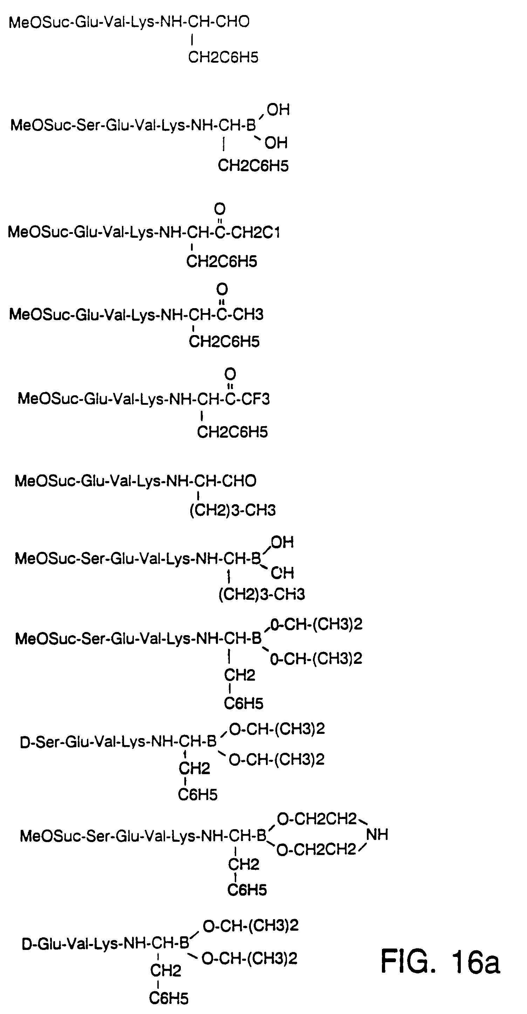

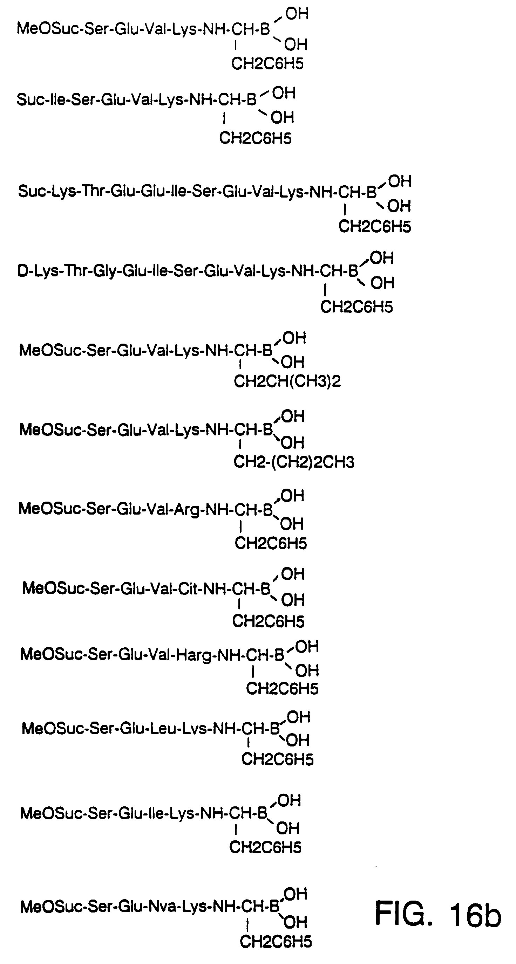

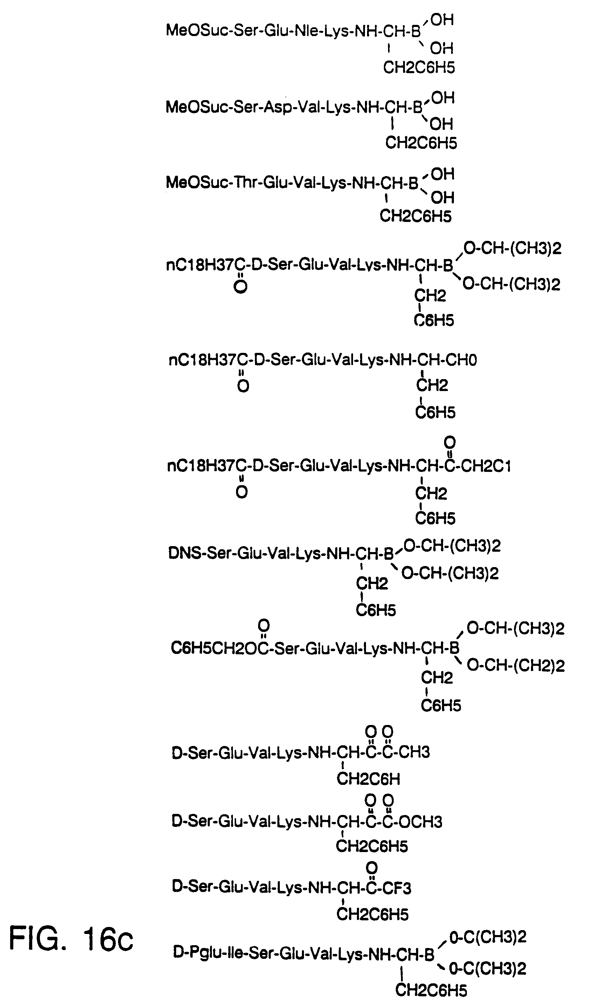

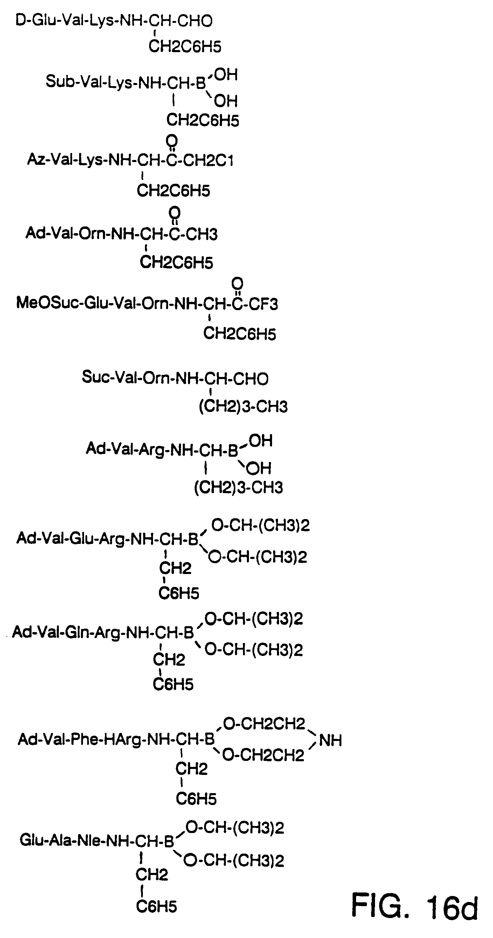

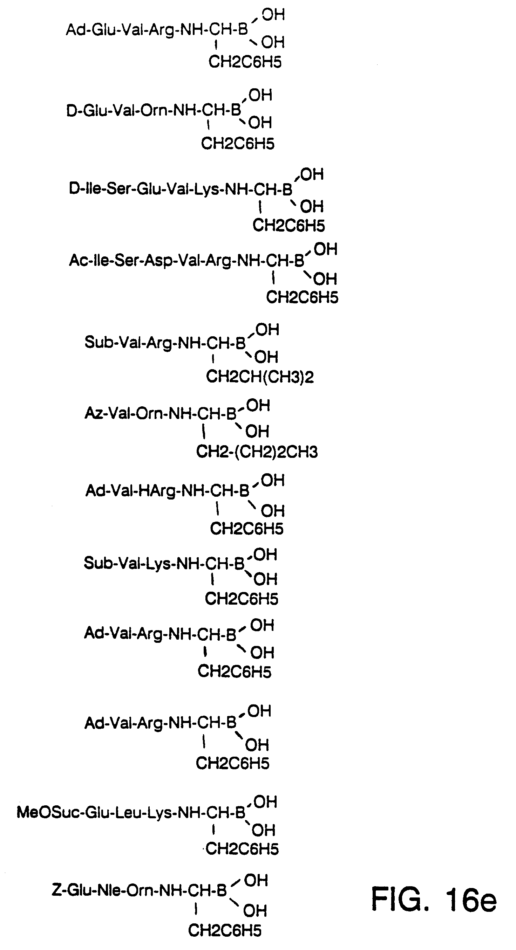

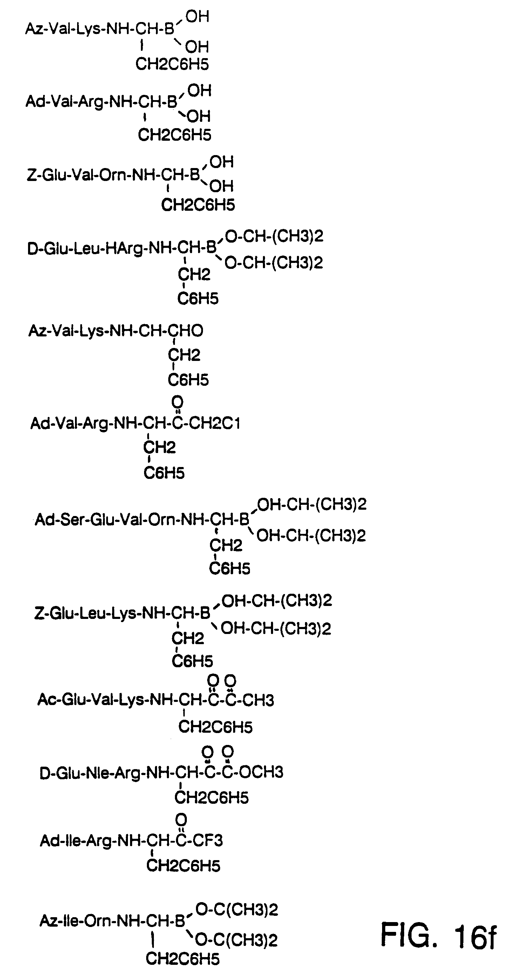

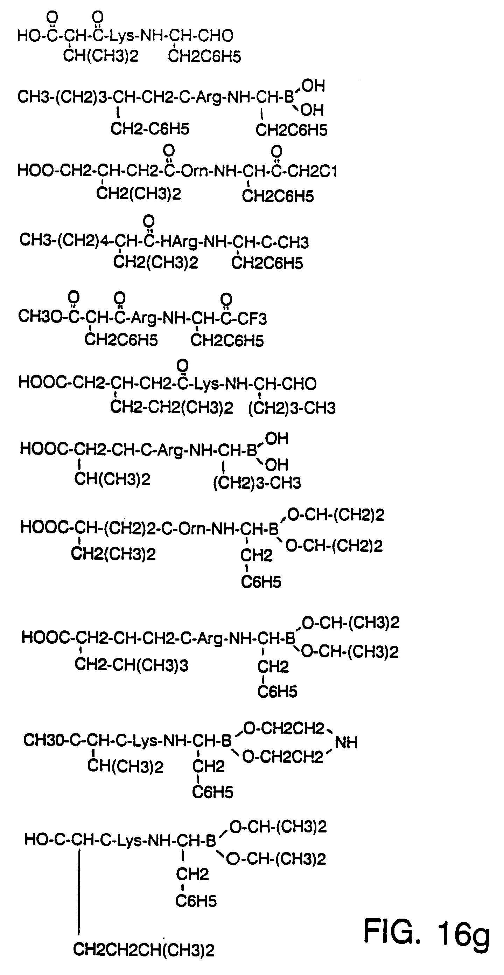

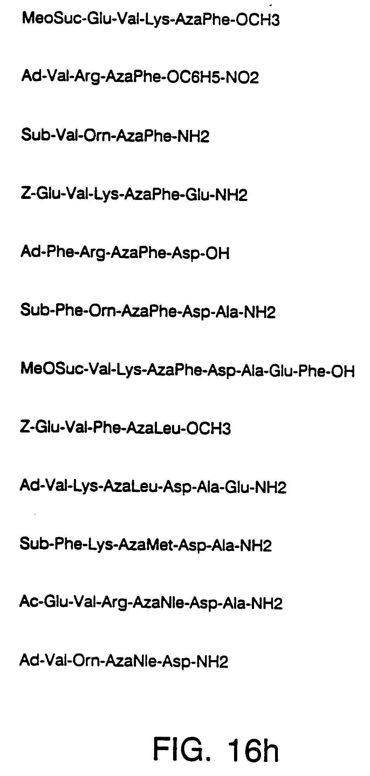

In addition to the inhibitors described in the examples, one skilled in the art may prepateother inhibitors of the enzymes of the invention using the above-described methods. A number of useful inhibitors are illustrated in Figure 16. The delineation of the enzymatic recognition requirements with the peptide substrates of the invention thus makes possible the design of inhibitors using similarly arranged peptide side chains. The removal of peptide bonds from the resulting molecules is, however, a desirable goal because the presence of such bonds may leave the inhibitor accessible to the attack of proteolytic enzymes. Additionally, such peptide bonds may affect the biological availability of the resulting therapeutic molecules. Several examples of the application of "depeptidization" to the P3 and P4 positions (nomenclature of Berger and Schechter, Phil. Trans. R. Soc. London, Ser. B (1970) 257:249) are given in the list of equivalents in Fig. 16. In principle, this approach may be employed at the P1 and P2 positions by the substitution of the amide bond -CONH- with the spatially similar -CH₂CH₂-, -CH₂-O-, -CH=CH- and -CH₂S-groups.

## Claims

1. A purified peptide having endopeptidase enzymatic activity which causes hydrolysis of a peptide bond between a methionine and an aspartic acid residue in a β-amyloid precursor protein, other than chymase or multicatalytic protease.

2. A method of detecting a protease indicative of Alzheimer's disease in the biological sample, the method comprising:
(a) providing a substrate for the protease which has the general formula:
R-A4-A3-A2-A1-R1,
wherein:
R represents a hydrogen atom or an N-terminal blocking group;
A4 represents a covalent bond, an amino acid or a peptide;
R1 represents a reporter group;
A3 represents a covalent bond, a D-amino acid, Phe, Tyr, Val, or a conservative amino acid substituent of Val;
A2 represents a hydrophobic amino acid, Lys or conservative amino acid substituent of Lys, and when A4 includes at least two amino acids A2 can represent any amino acid; and
A1 represents Met, Phe, Nle, Leu, Ile, Tyr or 3-phenylproline;
(b) contacting the biological sample with the substrate; and
(c) detecting cleavage of the substrate as an indication of the presence of the protease in the biological sample.

3. A method according to claim 2 wherein the biological sample is isolated from brain, liver, heart, muscle, white blood cells, mast cells, fibroblasts, serum, or cerebrospinal fluid.

4. A method according to claim 2 or 3 wherein the protease is chymase and/or multicatalytic protease.

5. A substrate for a protease having the general formula:
R-A4-A3-A2-A1-R1
wherein
R represents a hydrogen atom or a N-terminal blocking group;
A4 represents a covalent bond, an amino acid or a peptide;
R1 represents a reporter group;
A3 represents a covalent bond, a D-amino acid, Phe, Tyr, Val, or a conservative amino acid substituent of Val;
A2 represents a hydrophobic amino acid, Lys or a conservative amino acid substituent of Lys or, when A4 includes at least two amino acids, A2 can represent any amino acid; and
A1 represents Met, Phe, Nle, Leu, Ile, Tyr or 3-phenylproline.

6. A substrate according to claim 5 wherein:
(i) A3 represents Val, Nle, Nva, Ile, Leu, Tyr or Phe;
(ii) A2 represents Lys, Arg, Orn, Cit or Harg;
(iii) A4 represents Glu or Asp;
(iv) R represents Boc, Fmoc, Ac, Et, Suc, MeOSuc, Az, MeOAz, Ad, MeOAd, Sub, MeOSub, DNS, DNP, Z, or a D-amino acid; and/or
(v) R2 represents pNa or AMC.

7. A protease inhibitor having the general formula:
R-A4-A3-A2-Y
wherein
R represents a hydrogen atom or a N-terminal blocking group;
A4 represents a covalent bond, an amino acid or a peptide;
A3 represents a covalent bond, a D-amino acid, Phe, Tyr, Val, or a conservative amino acid substituent of Vla;
A2 represents a hydrophobic amino acid, lysine or a conservative amino acid substituent thereof or, when A4 includes at least two amino acids, A2 represents any amino acid; and
Y represents a group reactive with the active site of the protease.

8. A protease inhibitor according to claim 7 wherein Y has the formula 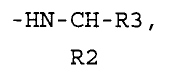 wherein
R2 is a lipophilic side chain substituent; and
R3 is a functional group which can react or interact with a hydroxyl group of the protease to form a transition-state analogue.

9. A protease inhibitor according to claim 7 or 8 wherein R2 represents a saturated or unsaturated alkyl or substituted alkyl group comprising two to ten carbon atoms or a alicyclic or aromatic group having five to ten carbon atoms or a arylalkyl group having eleven or fewer carbon atoms, such as R2 is an ethyl, n-propyl, isopropyl, n-butyl, or sec-butyl group.

10. A protease inhibitor according to any of claims 7 to 9 wherein R3 represents -CHO, -COCH₃, COCH₂Cl or COCH₂Br.

11. A protease inhibitor according to any of claims 7 to 10 wherein the protease is chymase and/or multicatalytic protease.

12. A method of purifying a protease according to claim 1, to at least 80% purity from a biological sample, the method comprising:
(a) homogenizing the biological sample in a low salt buffer including detergent;
(b) isolating the particulate fraction from the homogenized biological sample;
(c) dissolving the particulate fraction in a high salt buffer;
(d) isolating the soluble portion of the dissolved particulate fraction;
(e) binding the protease to an ion exchange or a heparin column and then eluting the protease from the ion exchange or heparin column; and
(f) binding the protease to a substrate affinity column and then eluting the protease from the substrate affinity column.

13. A method according to claim 12 wherein the biological sample is isolated from a mammal, such as a human, rat, cow, and/or pig.

14. A method according to claim 12 or 13 wherein the biological sample is isolated from the brain, liver, heart, muscle, white blood cells, mast cells or fibroblasts.

15. A method according to any of claims 12 to 14 wherein:
(i) the low salt comprises less than 0.5 M salt;
(ii) the detergent is ionic or non-ionic, for example Triton X-100;
(iii) the particulate fraction is separated from the homonogenized biological sample, and/or the soluble fraction is separated from the high salt, by centrifugation, such as between 30,000 to 3000,000 x g for 15 minutes to 120 minutes; and/or
(iv) the high salt is greater than 0.5 M salt.

16. A method according to any of claims 12 to 15 wherein the protease is bound to a heparin column and/or the affinity column comprises a substrate for the enzyme.

17. A method according to any of claims 12 to 16 wherein the substrate has the formula:
W-X-Y-Z
wherein
W is a covalent bond, an amino acid or a peptide;
X is valine or a conservative amino acid substituent thereof;
Y is s hydrophobic amino acid or Lys or a conservative substituent of Lys or, when W includes at least two amino acids, Y is any amino acid; and
Z represents Met, Phe, Nle, Ile, Leu, Tyr or 4-phenylproline.

18. The use of a protease inhibitor having the formula:
R-A4-A3-A2-Y
wherein
R represents a hydrogen atom or an N-terminal blocking group;
A4 represents a covalent bond, an amino acid or a peptide;
A3 represents a covalent bond, a D-amino acid, Phe, Tyr, Val, or a conservative amino acid substituent of Val;
A2 represents a hydrophobic amino acid or Lys or a conservative amino acid substituent thereof or, when A4 includes at least two amino acids, A2 represents any amino acid; and
Y is a group reactive with the active site of the protease;
in the preparation of an agent for treating alzheimer's disease.

19. The use according to claim 18 wherein the inhibitor is administered directly into the brain, or by an intramuscular, oral, or intranasal route.

20. A method of screening canditate chymase inhibitors, the method comprising:
(a) incubating a chymase substrate with one or more candidate chymase inhibitors and chymase; and
(b) determining whether any one or more of the candidate chymase inhibitors decrease the rate of cleavage of the substrate by the chymase.

21. A method of screening candidate multicatalytic protease inhibitors the method comprising:
(a) incubating a multicatalytic protease substrate with one or more candidate multicatalytic protease inhibitors and multicatalytic protease; and
(b) determining whether any one or more of the candidate multicatalytic protease inhibitors decrease the rate of cleavage of the substrate by the multicatalytic protease.

22. A method of screening candidate proteases capable of cleaving a β-amyloid precursor protein between Met and Asp, the method comprising:
(a) providing a peptide having the sequence EVKMDAEFRHDSGYEVHHQ or a substrate having the sequence:
R-Glu-Val-Lys-Met-R1,
wherein
R represents a hydrogen atom or a N-terminal blocking group;
R1 is a reporter group;
(b) contacting the candidate protease with the peptide or substrate, as appropriate; and
(c) determining whether the candidate protease cleaves the substrate.

23. The use of an inhibitor of chymase or an inhibitor of multicatalytic protease in the preparation of an agent for treating Alzheimer's disease.

24. A pharmaceutical composition comprising an inhibitor of chymase an/or an inhibitor of multicatalytic protease and a pharmaceutically acceptable carrier.

25. A composition according to claim 24 wherein the inhibitor is as claimed in any of claims 7 to 11.

26. A process for the preparation of a protease substrate having the general formula:
R-A4-A3-A2-A1-R1 I
wherein:
R represents a hydrogen atom or an N-terminal blocking group;
A4 represents a covalent bond, an amino acid or a peptide;
R1 represents a reporter group;
A3 represents a covalent bond, a D-amino acid, Phe, Tyr, Val, or a conservative amino acid substituent of Val;
A2 represents a hydrophobic amino acid, Lys or conservative amino acid substituent of Lys, and when A4 includes at least two amino acids A2 can represent any amino acid; and
A1 represents Met, Phe, Nle, Leu, Ile, Tyr or 3-phenylproline;
the process comprising reacting a precursor peptide of the formula:
R-A4-A3-A2-OH
with an amino acid derivative of the formula:
H-A1-R1
wherein R, A4, A3, A2, A1 and R1 are as defined above in formula I.

27. A process for the preparation of a protease inhibitor having the general formula:
R-A4-A3-A2-Y II
wherein:
R represents a hydrogen atom or a N-terminal blocking group;
A4 represents a covalent bond, an amino acid or a peptide;
A3 represents a covalent bond, a D-amino acid, Phe, Tyr, Val, or a conservative amino acid substituent of Vla;
A2 represents a hydrophobic amino acid, lysine or a conservative amino acid substituent thereof or, when A4 includes at least two amino acids, A2 represents any amino acid; and
Y represents a group reactive with the active site of the protease;
the process comprising reacting a precursor peptide of the formula:
R-A4-A3-A2-OH
with an amino acid derivative of the formula:
H-Y
wherein R, A4, A3, A2, A1 and R1 are as defined above in formula II.

28. A method of purifying a protease according to claim 1, from a biological tissue, the process comprising:
(a) homogenizing the tissue in a low salt buffer;
(b) isolating the soluble fraction from the homogenized tissue;
(c) binding the protease to, and then eluting it from, an ion exchange or heparin column;
(d) concentrating the protease and passing it through a gel filtration column; and
(e) binding the protease to, and then eluting it from, a hydroxyapatite or heparin column.

## Claims (Claims for the following Contracting State(s): ES)

1. A method of detecting a protease indicative of Alzheimer's disease in the biological sample, the method comprising:
(a) providing a substrate for the protease which has the general formula:
R-A4-A3-A2-A1-R1,
wherein:
R represents a hydrogen atom or an N-terminal blocking group;
A4 represents a covalent bond, an amino acid or a peptide;
R1 represents a reporter group;
A3 represents a covalent bond, a D-amino acid, Phe, Tyr, Val, or a conservative amino acid substituent of Val;
A2 represents a hydrophobic amino acid, Lys or conservative amino acid substituent of Lys, and when A4 includes at least two amino acids A2 can represent any amino acid; and
A1 represents Met, Phe, Nle, Leu, Ile, Tyr or 3-phenylproline;
(b) contacting the biological sample with the substrate; and
(c) detecting cleavage of the substrate as an indication of the presence of the protease in the biological sample.

2. A method according to claim 1 wherein the biological sample is isolated from brain, liver, heart, muscle, white blood cells, mast cells, fibroblasts, serum, or cerebrospinal fluid.

3. A method according to claim 1 or 2 wherein the protease is chymase and/or multicatalytic protease.

4. A process for the preparation of a protease substrate having the general formula:
R-A4-A3-A2-A1-R1 I
wherein:
R represents a hydrogen atom or an N-terminal blocking group;
A4 represents a covalent bond, an amino acid or a peptide;
R1 represents a reporter group;
A3 represents a covalent bond, a D-amino acid, Phe, Tyr, Val, or a conservative amino acid substituent of Val;
A2 represents a hydrophobic amino acid, Lys or conservative amino acid substituent of Lys, and when A4 includes at least two amino acids A2 can represent any amino acid; and
A1 represents Met, Phe, Nle, Leu, Ile, Tyr or 3-phenylproline;
the process comprising reacting a precursor peptide of the formula:
R-A4-A3-A2-OH
with an amino acid derivative of the formula:
H-A1-R1
wherein R, A4, A3, A2, A1 and R1 are as defined above in formula I.

5. A process according to claim 4 wherein:
(i) A3 represents Val, Nle, Nva, Ile, Leu, Tyr or Phe;
(ii) A2 represents Lys, Arg, Orn, Cit or Harg;
(iii) A4 represents Glu or Asp;
(iv) R represents Boc, Fmoc, Ac, Et, Suc, MeOSuc, Az, MeOAz, Ad, MeOAd, Sub, MeOSub, DNS, DNP, Z, or a D-amino acid; and/or
(v) R2 represents pNa or AMC.

6. A process for the preparation of a protease inhibitor having the general formula:
R-A4-A3-A2-Y II
wherein:
R represents a hydrogen atom or a N-terminal blocking group;
A4 represents a covalent bond, an amino acid or a peptide;
A3 represents a covalent bond, a D-amino acid, Phe, Tyr, Val, or a conservative amino acid substituent of Vla;
A2 represents a hydrophobic amino acid, lysine or a conservative amino acid substituent thereof or, when A4 includes at least two amino acids, A2 represents any amino acid; and
Y represents a group reactive with the active site of the protease;
the process comprising reacting a precursor peptide of the formula:
R-A4-A3-A2-OH
with an amino acid derivative of the formula:
H-Y
wherein R, A4, A3, A2, A1 and R1 are as defined above in formula II.

7. A process according to claim 6 wherein Y has the formula 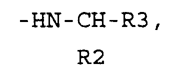 wherein
R2 is a lipophilic side chain substituent; and
R3 is a functional group which can react or interact with a hydroxyl group of the protease to form a transition-state analogue.

8. A process according to claim 6 or 7 wherein R2 represents a saturated or unsaturated alkyl or substituted alkyl group comprising two to ten carbon atoms or a alicyclic or aromatic group having five to ten carbon atoms or a arylalkyl group having eleven or fewer carbon atoms, such as R2 is an ethyl, n-propyl, isopropyl, n-butyl, or sec-butyl group.

9. A process according to any of claims 6 to 8 wherein R3 represents -CHO, -COCH₃, COCH₂Cl or COCH₂Br.

10. A process according to any of claims 6 to 9 wherein the protease is chymase and/or multicatalytic protease.

11. A method of purifying a protease having endopeptidase enzymatic activity which causes hydrolysis of a peptide bond between a methionine and an aspartic acid residue in a β-amyloid precursor protein, such as chymase or multicatalytic protease to at least 80% purity from a biological sample, the method comprising:
(a) homogenizing the biological sample in a low salt buffer including detergent;
(b) isolating the particulate fraction from the homogenized biological sample;
(c) dissolving the particulate fraction in a high salt buffer;
(d) isolating the soluble portion of the dissolved particulate fraction;
(e) binding the protease to an ion exchange or a heparin column and then eluting the protease from the ion exchange or heparin column; and
(f) binding the protease to a substrate affinity column and then eluting the protease from the substrate affinity column.

12. A method according to claim 11 wherein the biological sample is isolated from a mammal, such as a human, rat, cow, and/or pig.

13. A method according to claim 11 or 12 wherein the biological sample is isolated from the brain, liver, heart, muscle, white blood cells, mast cells or fibroblasts.

14. A method according to any of claims 11 to 13 wherein:
(i) the low salt comprises less than 0.5 M salt;
(ii) the detergent is ionic or non-ionic, for example Triton X-100;
(iii) the particulate fraction is separated from the homonogenized biological sample and/or the soluble fraction is separated from the high salt by centrifugation, such as between 30,000 to 3000,000 x g for 15 minutes to 120 minutes; and/or
(iv) the high salt is greater than 0.5 M salt.

15. A method according to any of claims 11 to 14 wherein the protease is bound to a heparin column and/or the affinity column comprises a substrate for the enzyme.

16. A method according to any of claims 11 to 15 wherein the substrate has the formula:
W-X-Y-Z
wherein
W is a covalent bond, an amino acid or a peptide;
X is valine or a conservative amino acid substituent thereof;
Y is s hydrophobic amino acid or Lys or a conservative substituent of Lys or, when W includes at least two amino acids, Y is any amino acid; and
Z represents Met, Phe, Nle, Ile, Leu, Tyr or 4-phenylproline.

17. A method according to any of claims 11 to 16 wherein the protease is chymase or multicatalytic protease.

18. The use of a protease inhibitor having the formula:
R-A4-A3-A2-Y
wherein
R represents a hydrogen atom or an N-terminal blocking group;
A4 represents a covalent bond, an amino acid or a peptide;
A3 represents a covalent bond, a D-amino acid, Phe, Tyr, Val, or a conservative amino acid substituent of Val;
A2 represents a hydrophobic amino acid or Lys or a conservative amino acid substituent thereof or, when A4 includes at least two amino acids, A2 represents any amino acid; and
Y is a group reactive with the active site of the protease;
in the preparation of an agent for treating Alzheimer's disease.

19. The use according to claim 18 wherein the inhibitor is administered directly into the brain, such as by an intramuscular, oral, or intranasal route.

20. A method of screening canditate chymase inhibitors, the method comprising:
(a) incubating a chymase substrate with one or more candidate chymase inhibitors and chymase; and
(b) determining whether any one or more of the candidate chymase inhibitors decrease the rate of cleavage of the substrate by the chymase.

21. A method of screening candidate multicatalytic protease inhibitors the method comprising:
(a) incubating a multicatalytic protease substrate with one or more candidate multicatalytic protease inhibitors and multicatalytic protease; and
(b) determining whether any one or more of the candidate multicatalytic protease inhibitors decrease the rate of cleavage of the substrate by the multicatalytic protease.

22. A method of screening candidate proteases capable of cleaving a β-amyloid precursor protein between Met and Asp, the method comprising:
(a) providing a peptide having the sequence EVKMDAEFRHDSGYEVHHQ or a substrate having the sequence:
R-Glu-Val-Lys-Met-R1,
wherein
R represents a hydrogen atom or a N-terminal blocking group;
R1 is a reporter group;
(b) contacting the candidate protease with the peptide or substrate, as appropriate; and
(c) determining whether the candidate protease cleaves the substrate.

23. The use of an inhibitor of chymase or an inhibitor of multicatalytic protease in the preparation of an agent for treating Alzheimer's disease.

24. A process for the preparation of a pharmaceutical composition, the process comprising admixing an inhibitor of chymase an/or an inhibitor of multicatalytic protease with a pharmaceutically acceptable carrier.

25. A process according to claim 24 wherein the inhibitor is prepared as claimed in any of claims 6 to 10.

26. A method of purifying a protease having endopeptidase enzymatic activity which causes hydrolysis of a peptide bond between a methionine and an aspartic acid residue in a β-amyloid precursor protein, such as chymase or multicatalytic protease from a biological tissue, the process comprising:
(a) homogenizing the tissue in a low salt buffer;
(b) isolating the soluble fraction from the homogenized tissue;
(c) binding the protease to, and then eluting it from, an ion exchange or heparin column;
(d) concentrating the protease and passing it through a gel filtration column; and
(e) binding the protease to, and then eluting it from, a hydroxyapatite or heparin column.

## Claims (Claims for the following Contracting State(s): GR)

1. A purified peptide having endopeptidase enzymatic activity which causes hydrolysis of a peptide bond between a methionine and an aspartic acid residue in a β-amyloid precursor protein, other than chymase or multicatalytic protease.

2. A method of detecting a protease indicative of Alzheimer's disease in the biological sample, the method comprising:
(a) providing a substrate for the protease which has the general formula:
R-A4-A3-A2-A1-R1,
wherein:
R represents a hydrogen atom or an N-terminal blocking group;
A4 represents a covalent bond, an amino acid or a peptide;
R1 represents a reporter group;
A3 represents a covalent bond, a D-amino acid, Phe, Tyr, Val, or a conservative amino acid substituent of Val;
A2 represents a hydrophobic amino acid, Lys or conservative amino acid substituent of Lys, and when A4 includes at least two amino acids A2 can represent any amino acid; and
A1 represents Met, Phe, Nle, Leu, Ile, Tyr or 3-phenylproline;
(b) contacting the biological sample with the substrate; and
(c) detecting cleavage of the substrate as an indication of the presence of the protease in the biological sample.

3. A method according to claim 2 wherein the biological sample is isolated from brain, liver, heart, muscle, white blood cells, mast cells, fibroblasts, serum, or cerebrospinal fluid.

4. A method according to claim 2 or 3 wherein the protease is chymase and/or multicatalytic protease.

5. A substrate for a protease having the general formula:
R-A4-A3-A2-A1-R1
wherein
R represents a hydrogen atom or a N-terminal blocking group;
A4 represents a covalent bond, an amino acid or a peptide;
R1 represents a reporter group;
A3 represents a covalent bond, a D-amino acid, Phe, Tyr, Val, or a conservative amino acid substituent of Val;
A2 represents a hydrophobic amino acid, Lys or a conservative amino acid substituent of Lys or, when A4 includes at least two amino acids, A2 can represent any amino acid; and
A1 represents Met, Phe, Nle, Leu, Ile, Tyr or 3-phenylproline.

6. A substrate according to claim 5 wherein:
(i) A3 represents Val, Nle, Nva, Ile, Leu, Tyr or Phe;
(ii) A2 represents Lys, Arg, Orn, Cit or Harg;
(iii) A4 represents Glu or Asp;
(iv) R represents Boc, Fmoc, Ac, Et, Suc, MeOSuc, Az, MeOAz, Ad, MeOAd, Sub, MeOSub, DNS, DNP, Z, or a D-amino acid; and/or
(v) R2 represents pNa or AMC.

7. A process for the preparation of a protease inhibitor having the general formula:
R-A4-A3-A2-Y II
wherein:
R represents a hydrogen atom or a N-terminal blocking group;
A4 represents a covalent bond, an amino acid or a peptide;
A3 represents a covalent bond, a D-amino acid, Phe, Tyr, Val, or a conservative amino acid substituent of Vla;
A2 represents a hydrophobic amino acid, lysine or a conservative amino acid substituent thereof or, when A4 includes at least two amino acids, A2 represents any amino acid; and
Y represents a group reactive with the active site of the protease;
the process comprising reacting a precursor peptide of the formula:
R-A4-A3-A2-OH
with an amino acid derivative of the formula:
H-Y
wherein R, A4, A3, A2, A1 and R1 are as defined above in formula II.

8. A process according to claim 7 wherein Y has the formula 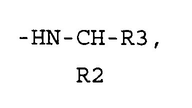 wherein
R2 is a lipophilic side chain substituent; and
R3 is a functional group which can react or interact with a hydroxyl group of the protease to form a transition-state analogue.

9. A process according to claim 7 or 8 wherein R2 represents a saturated or unsaturated alkyl or substituted alkyl group comprising two to ten carbon atoms or a alicyclic or aromatic group having five to ten carbon atoms or a arylalkyl group having eleven or fewer carbon atoms, such as R2 is an ethyl, n-propyl, isopropyl, n-butyl, or sec-butyl group.

10. A process according to any of claims 7 to 9 wherein R3 represents -CHO, -COCH₃, COCH₂Cl or COCH₂Br.

11. A process according to any of claims 7 to 10 wherein the protease is chymase and/or multicatalytic protease.

12. A method of purifying a protease according to claim 1, to at least 80% purity from a biological sample, the method comprising:
(a) homogenizing the biological sample in a low salt buffer including detergent;
(b) isolating the particulate fraction from the homogenized biological sample;
(c) dissolving the particulate fraction in a high salt buffer;
(d) isolating the soluble portion of the dissolved particulate fraction;
(e) binding the protease to an ion exchange or a heparin column and then eluting the protease from the ion exchange or heparin column; and
(f) binding the protease to a substrate affinity column and then eluting the protease from the substrate affinity column.

13. A method according to claim 12 wherein the biological sample is isolated from a mammal, such as a human, rat, cow, and/or pig.

14. A method according to claim 12 or 13 wherein the biological sample is isolated from the brain, liver, heart, muscle, white blood cells, mast cells or fibroblasts.

15. A method according to any of claims 12 to 14 wherein:
(i) the low salt comprises less than 0.5 M salt;
(ii) the detergent is ionic or non-ionic, for example Triton X-100;
(iii) the particulate fraction is separated from the homonogenized biological sample and/or the soluble fraction is separated from the high salt by centrifugation, such as between 30,000 to 3000,000 x g for 15 minutes to 120 minutes; and/or
(iv) the high salt is greater than 0.5 M salt.

16. A method according to any of claims 12 to 15 wherein the protease is bound to a heparin column and/or the affinity column comprises a substrate for the enzyme.

17. A method according to any of claims 12 to 16 wherein the substrate has the formula:
W-X-Y-Z
wherein
W is a covalent bond, an amino acid or a peptide;
X is valine or a conservative amino acid substituent thereof;
Y is s hydrophobic amino acid or Lys or a conservative substituent of Lys or, when W includes at least two amino acids, Y is any amino acid; and
Z represents Met, Phe, Nle, Ile, Leu, Tyr or 4-phenylproline.

18. The use of a protease inhibitor having the formula:
R-A4-A3-A2-Y
wherein
R represents a hydrogen atom or an N-terminal blocking group;
A4 represents a covalent bond, an amino acid or a peptide;
A3 represents a covalent bond, a D-amino acid, Phe, Tyr, Val, or a conservative amino acid substituent of Val;
A2 represents a hydrophobic amino acid or Lys or a conservative amino acid substituent thereof or, when A4 includes at least two amino acids, A2 represents any amino acid; and
Y is a group reactive with the active site of the protease;
in the preparation of an agent for treating Alzheimer's disease.

19. The use according to claim 18 wherein the inhibitor is administered directly into the brain, such as by an intramuscular, oral, or intranasal route.

20. A method of screening canditate chymase inhibitors, the method comprising:
(a) incubating a chymase substrate with one or more candidate chymase inhibitors and chymase; and
(b) determining whether any one or more of the candidate chymase inhibitors decrease the rate of cleavage of the substrate by the chymase.

21. A method of screening candidate multicatalytic protease inhibitors the method comprising:
(a) incubating a multicatalytic protease substrate with one or more candidate multicatalytic protease inhibitors and multicatalytic protease; and
(b) determining whether any one or more of the candidate multicatalytic protease inhibitors decrease the rate of cleavage of the substrate by the multicatalytic protease.

22. A method of screening candidate proteases capable of cleaving a β-amyloid precursor protein between Met and Asp, the method comprising:
(a) providing a peptide having the sequence EVKMDAEFRHDSGYEVHHQ or a substrate having the sequence:
R-Glu-Val-Lys-Met-R1,
wherein
R represents a hydrogen atom or a N-terminal blocking group;
R1 is a reporter group;
(b) contacting the candidate protease with the peptide or substrate, as appropriate; and
(c) determining whether the candidate protease cleaves the substrate.

23. The use of an inhibitor of chymase or an inhibitor of multicatalytic protease in the preparation of an agent for treating Alzheimer's disease.

24. A process for the preparation of a pharmaceutical composition, the process comprising admixing an inhibitor of chymase an/or an inhibitor of multicatalytic protease with a pharmaceutically acceptable carrier.

25. A process according to claim 24 wherein the inhibitor is as claimed in any of claims 7 to 11.

26. A process for the preparation of a protease substrate having the general formula:
R-A4-A3-A2-A1-R1 I
wherein:
R represents a hydrogen atom or an N-terminal blocking group;
A4 represents a covalent bond, an amino acid or a peptide;
R1 represents a reporter group;
A3 represents a covalent bond, a D-amino acid, Phe, Tyr, Val, or a conservative amino acid substituent of Val;
A2 represents a hydrophobic amino acid, Lys or conservative amino acid substituent of Lys, and when A4 includes at least two amino acids A2 can represent any amino acid; and
A1 represents Met, Phe, Nle, Leu, Ile, Tyr or 3-phenylproline;
the process comprising reacting a precursor peptide of the formula:
R-A4-A3-A2-OH
with an amino acid derivative of the formula:
H-A1-R1
wherein R, A4, A3, A2, A1 and R1 are as defined above in formula I.

27. A method of purifying a protease according to claim 1, from a biological tissue, the process comprising:
(a) homogenizing the tissue in a low salt buffer;
(b) isolating the soluble fraction from the homogenized tissue;
(c) binding the protease to, and then eluting it from, an ion exchange or heparin column;
(d) concentrating the protease and passing it through a gel filtration column; and
(e) binding the protease to, and then eluting it from, a hydroxyapatite or heparin column.
